(19) Europäisches Patentamt
European Patent Office
Office européen des brevets

(11) Veröffentlichungsnummer : **0 031 951**
**B1**

(12) # EUROPÄISCHE PATENTSCHRIFT

(45) Veröffentlichungstag der Patentschrift : **25.07.84**

(21) Anmeldenummer : **80108188.6**

(22) Anmeldetag : **29.12.80**

(51) Int. Cl.[3] : **C 07 D499/68**, C 07 D499/70, A 61 K 31/43// C07C133/00, C07D333/24

(54) **Formylderivate von Hydrazinomethylpenicillinen, Verfahren zu ihrer Herstellung und diese Verbindungen enthaltende Arzneimittel.**

(30) Priorität : **27.12.79 IT 2839979**

(43) Veröffentlichungstag der Anmeldung : **15.07.81 Patentblatt 81/28**

(45) Bekanntmachung des Hinweises auf die Patenterteilung : **25.07.84 Patentblatt 84/30**

(84) Benannte Vertragsstaaten : **AT BE CH DE FR GB IT LI LU NL SE**

(56) Entgegenhaltungen :
**GB-A- 1 048 907**
**GB-A- 1 509 200**
**GB-A- 1 524 950**
**US-A- 3 174 964**

(73) Patentinhaber : **CRAF Sud**
**Via di Fossignano, 2**
**Aprilia Latina (Roma) (IT)**

(72) Erfinder : **Bolis, Goffredo**
**v. Roma 49**
**Antegnate (Bergamo) (IT)**
Erfinder : **Giani, Roberto**
**v. G. Borsi 4**
**Milano (IT)**
Erfinder : **Pifferi, Giorgio**
**v. Carlone 8**
**Milano (IT)**
Erfinder : **Broccali, Giampietro, Dr.**
**v. Indipendenza 20**
**Milano (IT)**
Erfinder : **Pinza, Mario**
**v. per Cesano Boscone 32**
**Corsico (Milano) (IT)**

(74) Vertreter : **Beszédes, Stephan G., Dr. et al**
**Am Heideweg 2 Postfach 1168**
**D-8060 Dachau (DE)**

**EP 0 031 951 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue Formylderivate von Hydrazinomethylpenicillinen, ein Verfahren zu ihrer Herstellung und diese Verbindungen enthaltende Arzneimittel, insbesondere solche mit antibakterieller beziehungsweise baktericider Wirkung.

In der deutschen Offenlegungsschrift 23 55 740 sind α-Hydrazinobenzylpenicilline der allgemeinen Formel

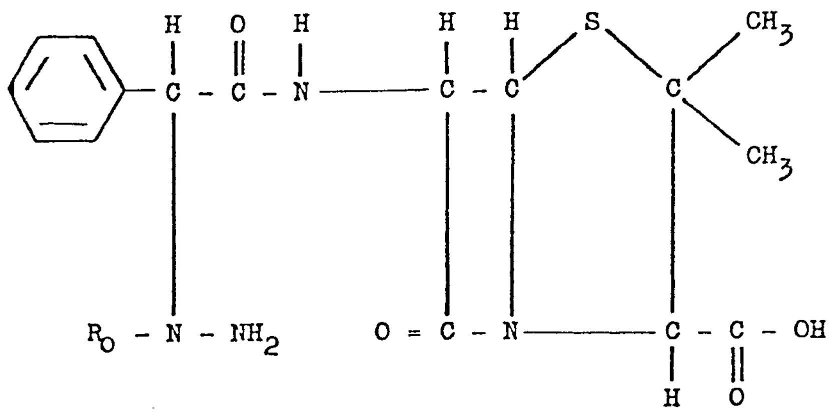

O

worin

$R_0$ für Wasserstoff oder einen geradkettigen oder verzweigten niederen Alkylrest mit 1 bis 6 Kohlenstoffatomen steht,

in Form von Racematen oder Enantiomeren sowie ihre nichttoxischen Salze und leicht hydrolysierbaren Ester beschrieben.

Ferner sind aus der britischen Patentschrift 1 524 950 α-Hydrazinoacetamidopenicilline der allgemeinen Formel

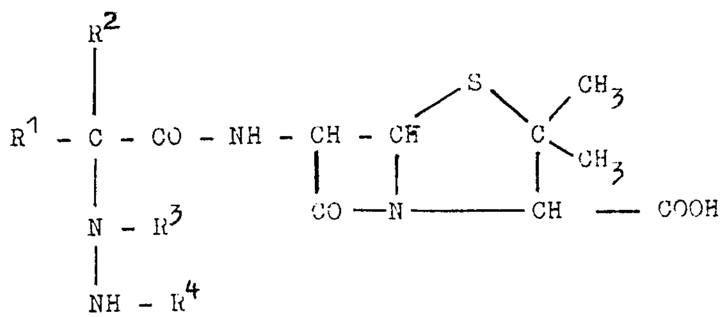

Oa

worin

$R^1$ unter anderem für einen Thernyl- oder Phenylrest stehen kann,

$R^2$ unter anderem Wasserstoff bedeuten kann und

$R^3$ und $R^4$ unter anderem Wasserstoff oder Alkylcarbonyl-, Alkoxycarbonyl-, Arylcarbonyl-, Aryloxycarbonyl-, Aralkylcarbonyl- beziehungsweise substituierte oder nicht substituierte Aralkoxycarbonylreste darstellen können,

sowie ihre pharmazeutisch brauchbaren Salze und Ester bekannt. $R^3$ und $R^4$ können aber keine Formylreste, welche auch nicht unter die Definition « Alkylcarbonylreste » fallen, sein. Zwar wird für die Verbindungen der britischen Patentschrift 1 524 950 eine antibakterielle Wirksamkeit behauptet, diese liegt aber in Wirklichkeit nicht vor. Dazu ist zu bemerken, daß in Tetrahedron Letters No. 41 (1976), Seiten 3 731 bis 3 734 in einem Artikel der Inhaberin des britischen Patentes 1 524 950 angegeben ist, daß es 6β-[α-($N^β$-p-nitrobenzyloxycarbonylhydrazino)-phenylacetamido]-penicillansäure {Verbindung des Beispieles 3 der britischen Patentschrift 1 524 950} an einer brauchbaren mikrobiologischen Wirksamkeit mangelt. Als Verfahren zur Herstellung der Verbindungen der britischen Patentschrift 1 524 950 ist in dieser auch das Acylieren von geeigneten α-Hydrazinoacetamidopenicillinsalzen, bei welchen die Hydrazinstickstoffatome Wasserstoff aufweisen, beispielsweise mit Alkylcarbonsäuren oder Aralkylcarbonsäuren oder das Umsetzen von 6-Aminopenicillansäure mit geeigneten α-Hydrazinoessigsäurederivaten angegeben.

Ähnliche Verbindungen sind aus der britischen Patentschrift 1 509 200 bekannt. Bei diesen ist aber an das Stickstoffatom 2 zwingend ein substituierter Alkylidenrest gebunden.

Weiterhin sind durch die US-Patentschrift 3 174 964 6β-Aminopenicillansäurederivate, bei welchen

die 6-Aminogruppe unter anderem durch einen, gegebenenfalls durch einen Carboxhydrazidorest substituierten, Phenylacetylrest oder einen Thienylrest unter Amidbildung substituiert sein kann, umfaßt, wobei auch ihre antibiotische Wirksamkeit erwähnt ist, jedoch ohne nähere zahlenmäßige Angaben.

Außerdem sind in der britischen Patentschrift 1 048 907 Formamidomethylpenicilline der allgemeinen Formel

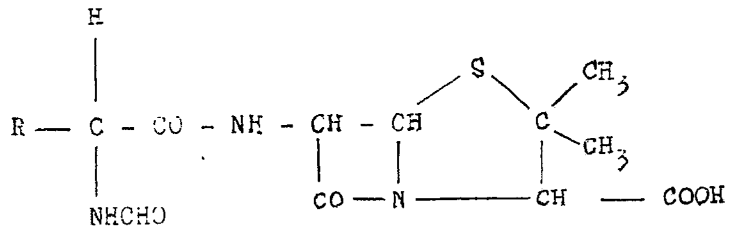

Ob

worin R für Wasserstoff oder einen Alkylrest, Aralkylrest, Arylrest oder heterocyclischen Rest, welcher substituiert sein kann, steht, sowie ihre nicht toxischen Salze beschrieben. Auch ist ihre antibakterielle Wirksamkeit erwähnt, jedoch ohne nähere zahlenmäßige Angaben.

Der Erfindung liegt die Aufgabe zugrunde, überlegene pharmakologische Wirkungen aufweisende neue Formylderivate von Hydrazinomethylpenicillinen, ein Verfahren zur Herstellung derselben und diese Verbindungen enthaltende Arzneimittel zu schaffen.

Dies wurde überraschenderweise durch die Erfindung erreicht.

Es wurde nämlich überraschenderweise festgestellt, daß durch Ersatz des Wasserstoffatomes beziehungsweise der Wasserstoffatome am Stickstoffatom beziehungsweise an den Stickstoffatomen in der beziehungsweise den 1- und/oder 2-Stellung(en) der Hydrazinogruppe der aus der deutschen Offenlegungsschrift 23 55 740 bekannten Verbindungen neue Penicilline mit wertvoller antibakterieller beziehungsweise baktericider Wirksamkeit erhalten werden. Ferner wurde überraschenderweise festgestellt, daß der Ersatz der Phenylgruppe durch die heterocyclische Thienylgruppe eine weitere Bereicherung hinsichtlich der antibakteriellen beziehungsweise baktericiden Wirksamkeit mit sich bringt.

Gegenstand der Erfindung sind daher Formylderivate von Hydrazinomethylpenicillinen der allgemeinen Formel

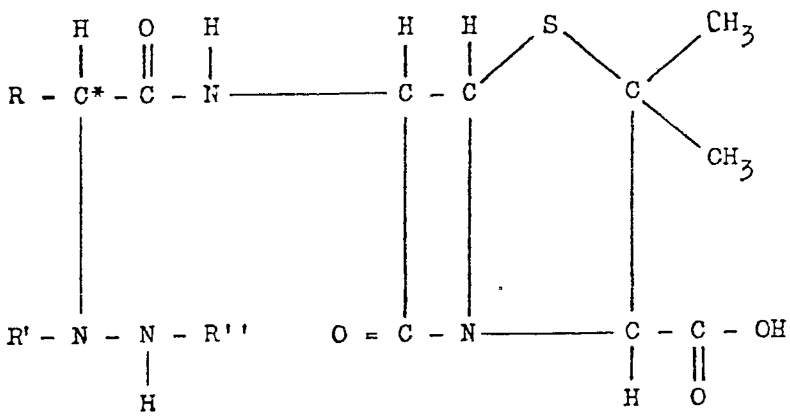

(I)

worin

R für eine Thienyl- oder Phenylgruppe steht,

R′ Wasserstoff, eine Formylgruppe oder eine Alkylgruppe mit 1 bis 3 Kohlenstoffatomen bedeutet,

R″ Wasserstoff oder eine Formylgruppe darstellt und

das Sternchen (*) ein Asymmetriezentrum des Moleküles darstellt,

mit der Maßgabe, daß mindestens eines der Symbole von R′ und R″ eine Formylgruppe darstellt, in Form der getrennten Epimere oder von Mischungen derselben sowie ihre Salze und 1-(Äthoxycarbonyloxy)-äthyl- und Pivaloyloxymethylester. (Die Symbole in der obigen allgemeinen Formel I entsprechen nicht stets den in den erörterten Druckschriften verwendeten).

Die erfindungsgemäßen Formylderivative von Hydrazinomethylpenicillinen unterscheiden sich von sämtlichen bekannten Verbindungen darin, daß ihre an die 6-Aminogruppe des Penicillansäurerestes unter Amidbildung gebundene substituierte Acetylgruppe durch eine Formylhydrazino- oder Diformylhydrazinogruppe substituiert ist.

So ist bei den erfindungsgemäßen Verbindungen mindestens eines der Symbole von R′ und R″ eine Formylgruppe, während dies bei den Verbindungen der britischen Patentschrift 1 524 950 nicht vorgesehen ist. Im Gegensatz zur nicht brauchbaren antibakteriellen Wirksamkeit der Verbindungen der britischen Patentschrift 1 524 950 haben die erfindungsgemäßen Verbindungen eine überlegene antibakterielle beziehungsweise baktericide Wirksamkeit.

Noch größer ist der Unterschied der erfindungsgemäßen Verbindungen zu denen der britischen Patentschrift 1 509 200, da in den erfindungsgemäßen Verbindungen auch noch im Gegensatz zur zwingenden Festlegung einer substituierten Alkylidengruppe am Stickstoffatom 2 der Hydrazinogruppe in der britischen Patentschrift 1 509 200 keine solche vorliegen kann.

Der wesentliche Unterschied zwischen den erfindungsgemäßen Verbindungen und denen der US-Patentschrift 3 174 964 besteht darin, daß die ersteren eine Formylhydrazino- oder Diformylhydrazinogruppe aufweisen, während eine solche bei den Verbindungen der genannten Druckschrift nicht vorgesehen ist, wobei zu bemerken ist, daß dies auch dann gilt, wenn bei den Verbindungen der genannten Druckschrift die 6-Aminogruppe durch eine Carboxhydrazidophenylacetylgruppe substituiert ist, denn die Carboxhydrazidogruppe oder anders ausgedrückt Hydrazinocarbonylgruppe mit der Formel

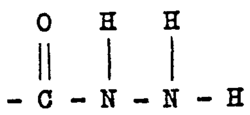

Oc

weist keine Formylgruppe auf. Im Falle der erfindungsgemäßen Verbindungen, bei welchen R für eine Thienylgruppe steht, kommt als weiterer Unterschied zu den Verbindungen der US-Patentschrift 3 174 964 hinzu, daß bei den erfindungsgemäßen Verbindungen eine, wie angegeben, substituierte Thienylmethylcarbonylgruppe an der 6-Aminogruppe des Penicillanrestes hängt, während bei den Verbindungen der US-Patentschrift 3 174 964 an deren Stelle allenfalls eine Thienylgruppe stehen kann. Hinzukommt nocht, daß von der enormen Zahl der in der genannten Druckschrift erwähnten Verbindungen keine den erfindungsgemäßen Verbindungen selbst entfernt ähnlich ist.

Von den Verbindungen der britischen Patentschrift 1 048 907 unterscheiden sich die erfindungsgemäßen Verbindungen grundlegend darin, daß sie statt der Formamidogruppe eine Formylhydrazino- oder Diformylhydrazinogruppe aufweisen. Die letzteren haben gegen viele Bakterien eine bessere Wirkung als die ersteren.

Vorzugsweise ist die Alkylgruppe, die für R' stehen kann, eine solche mit 1 oder 2, insbesondere 1 Kohlenstoffatom(en).

Es ist auch bevorzugt, daß die Thienylgruppe, die für R stehen kann, eine Thien-2-ylgruppe ist.

Als Salze der Formylderivate von Hydrazinomethylpenicillinen der allgemeinen Formel I kommen die pharmazeutisch brauchbaren in Frage. Bevorzugte Salze der Formylderivate von Hydrazinomethylpenicillinen der allgemeinen Formel I sind solche mit Alkalimetallen, insbesondere Natrium oder Kalium, Erdalkalimetallen, wie Calcium, Ammoniak, Aminen, insbesondere Triäthylamin, Procain, Dibenzylamin oder N,N'-Dibenzyläthylendiamin, oder anorganischen oder organischen Säuren, insbesondere Salzsäure, Bromwasserstoffsäure, Jodwasserstoffsäure, Schwefelsäure, Phosphorsäure, Essigsäure, Bernsteinsäure, Weinsäure oder Ascorbinsäure.

Besonders bevorzugte erfindungsgemäße Verbindungen sind (R)-6β-[α-(2'-Formyl-1'-methylhydrazino)-phenyl-acetamido]-penicillansäure, (R)-6β-[α-(1',2'-Diformylhydrazino)-phenylacetamido]-penicillansäure, (R)-6β-[α-[2'-Formylhydrazino)-phenylacetamido]-penicillansäure und (R)-6β-[α-(2'-Formylhydrazino)-phenylacetamido]-penicillansäure-{1"-(äthoxycarbonyloxy)-äthyl}-ester.

Gegenstand der Erfindung ist auch ein Verfahren zur Herstellung der erfindungsgemäßen Verbindungen der allgemeinen Formel I, welches dadurch gekennzeichnet ist, daß in an sich bekannter Weise

a) Hydrazinomethylpenicilline der allgemeinen Formel

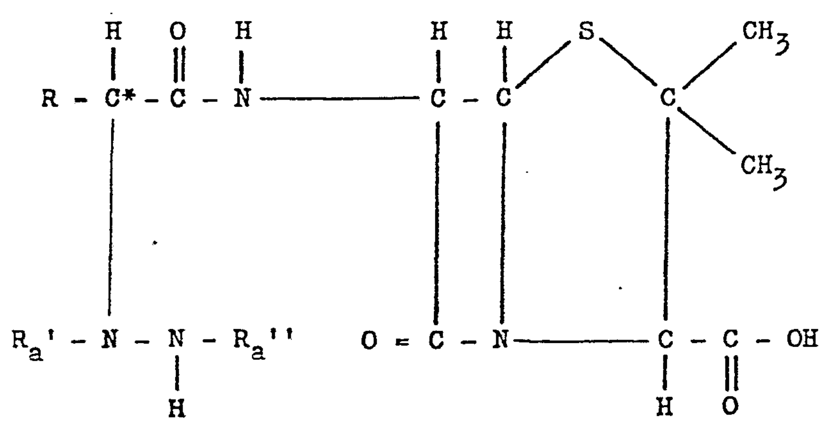

(II)

worin

R und das Sternchen (*) wie oben festgelegt sind,

$R_a'$ für Wasserstoff, eine Alkylgruppe mit 1 bis 3 Kohlenstoffatomen oder eine übliche Schutzgruppe steht und

$R_a''$ Wasserstoff oder eine übliche Schutzgruppe darstellt,

wobei mindestens eines der symbole von $R_a'$ und $R_a''$ keine Schutzgruppe bedeutet, in Form der getrennten Epimere oder von Mischungen derselben beziehungsweise Salze oder Ester derselben, gegebenenfalls nur mit Schutzgruppenfunktion der Estergruppe, unter wasserfreien Bedingungen mit dem gemischten Anhydrid von Essigsäure und Ameisensäure formyliert werden und die gegebenenfalls vorliegende(n) Schutzgruppe(n) entfernt wird beziehungsweise werden oder

b) α-Hydrazinoessigsäurederivate der allgemeinen Formel

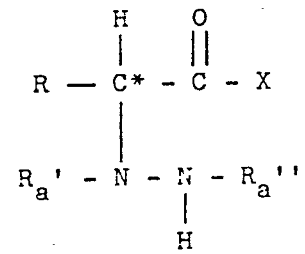

(III)

worin

R und das Sternchen (*) wie oben festgelegt sind,

$R_a'$ für Wasserstoff, eine Alkylgruppe mit 1 bis 3 Kohlenstoffatomen oder eine übliche Schutzgruppe steht,

$R_a''$ Wasserstoff oder eine übliche Schutzgruppe bedeutet,

wobei mindestens eines der Symbole von $R_a'$ und $R_a''$ keine Schutzgruppe bedeutet, und

X eine Hydroxygruppe oder eine bei der anschließenden Kondensation oder vor dieser in sonstiger üblicher Weise abspaltbare übliche Schutzgruppe darstellt,

in Form der getrennten Epimere oder von Mischungen derselben, gegebenenfalls als Salz, mit dem gemischten Anhydrid von Essigsäure und Ameisensäure unter wasserfreien Bedingungen formyliert werden und die so erhaltenen Formylderivate von α-Hydrazino-essigsäurederivaten der allgemeinen Formel

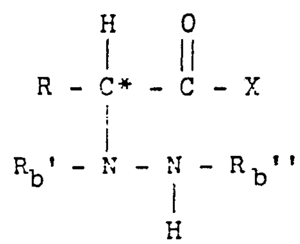

(IV)

worin

R und das Sternchen (*) wie oben festgelegt sind,

$R_b'$ für Wasserstoff, eine Formylgruppe, eine Alkylgruppe mit 1 bis 3 Kohlenstoffatomen oder eine übliche Schutzgruppe steht,

$R_b''$ Wasserstoff, eine Formylgruppe oder eine übliche Schutzgruppe bedeutet,

wobei mindestens eines der Symbole von $R_b'$ und $R_b''$ keine Schutzgruppe bedeutet und mindestens eines der Symbole von $R_b'$ und $R_b''$ eine Formylgruppe darstellt, und

X wie vorstehend festgelegt ist,

gegebenenfalls nach ihrer Überführung in Salze, mit 6-Aminopenicillansäure als solcher oder als Salz oder in Form von Estern, gegebenenfalls nur mit üblicher Schutzgruppenfunktion der Estergruppe, kondensiert werden sowie die gegebenenfalls vorliegende(n) Schutzgruppe(n) vor und/oder nach dieser Umsetzung entfernt wird beziehungsweise werden oder

c) α-Hydrazinoessigsäurederivate der allgemeinen Formel

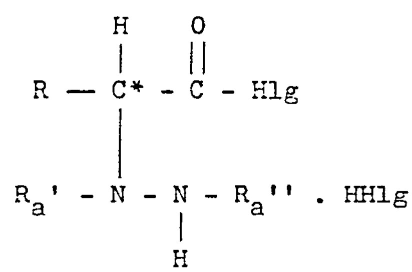

(V)

worin

R und das Sternchen (*) wie oben festgelegt sind,

$R_a'$ für Wasserstoff, eine Alkylgruppe mit 1 bis 3 Kohlenstoffatomen oder eine übliche Schutzgruppe steht,

$R_a''$ Wasserstoff oder eine übliche Schutzgruppe bedeutet,

wobei mindestens eines der Symbole von $R_a'$ und $R_a''$ keine Schutzgruppe bedeutet, und

Hlg für Chlor oder Brom steht,

in Form der getrennten Epimere oder von Mischungen derselben, mit 6-Aminopenicillansäure als solcher oder als Salz oder in Form von Estern, gegebenenfalls nur mit üblicher Schutzgruppenfunktion der Estergruppe, kondensiert werden und die so erhaltenen Hydrazinomethylpenicilline der allgemeinen Formel

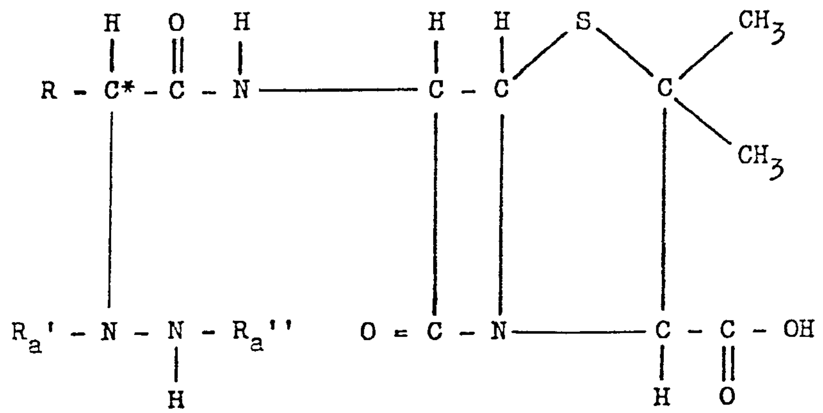

(II)

worin R, das Sternchen (*), $R_a'$ und $R_a''$ wie vorstehend festgelegt sind, mit dem gemischten Anhydrid von Essigsäure und Ameisensäure unter wasserfreien Bedingungen formyliert werden sowie die gegebenenfalls vorliegende(n) Schutzgruppe(n) entfernt wird beziehungsweise werden,

worauf in an sich bekannter Weise gegebenenfalls die so erhaltenen Formylderivate von Hydrazinomethylpenicillinen der allgemeinen Formel I mit Säuren in pharmazeutisch brauchbare Säureadditionssalze überführt oder verestert werden beziehungsweise gegebenenfalls die so erhaltenen Säureadditionssalze der Formylderivate von Hydrazinomethylpenicillinen der allgemeinen Formel I in die freien Formylderivate von Hydrazinomethylpenicillinen der allgemeinen Formel I oder in andere pharmazeutisch brauchbare Salze überführt werden beziehungsweise gegebenenfalls die so erhaltenen Ester der Formylderivate von Hydrazinomethylpenicillinen der allgemeinen Formel I in die freien Formylderivate von Hydrazinomethylpenicillinen der allgemeinen Formel I überführt werden und/oder gegebenenfalls die so erhaltenen Mischungen der Epimere der Formylderivate von Hydrazinomethylpenicillinen der allgemeinen Formel I in die Epimere gespalten beziehungsweise die so erhaltenen Epimere der Formylderivate von Hydrazinomethylpenicillinen der allgemeinen Formel I racemisiert werden.

Die Formylierung wird vorteilhaft in organischen Lösungsmitteln für die Reaktionsteilnehmer, wie tertiären organischen Basen, beispielsweise tertiären Aminen, oder, aprotischen Lösungsmitteln, z. B. Methylenchlorid, durchgeführt.

Als übliche Schutzgruppen an der Hydrazinogruppe, für die $R_a'$ oder $R_a''$ beziehungsweise $R_b'$ oder $R_b''$ stehen, kann beziehungsweise können, können vorteilhaft die Nitrobenzyloxycarbonylgruppe und die Benzyloxycarbonylgruppe, eingesetzt werden. Diese können zweckmäßig dann, wenn die Formylgruppe nur am 1 Stickstoffatom der Hydrazinogruppe einzuführen ist, am anderen Stickstoffatom verwendet werden.

Als Ester der 6-β-Aminopenicillansäure können der Pivaloyloxymethylester und der 1-(Äthoxycarbonyloxy)-äthylester, sowie ferner Trialkylsilylester, insbesondere der Trimethylsilylester, aber auch die Triäthylsilyl- und Tri-n-butylsilylester, und Nitrobenzylester, insbesondere der p-Nitrobenzylester, eingesetzt werden.

Als Salze der 6-β-Aminopenicillansäure können vorteilhaft die Trialkylaminsalze, insbesondere das Trimethylaminsalz, eingesetzt werden.

In den bei der Variante b) des erfindungsgemäßen Verfahrens als Ausgangsstoffe verwendeten α-Hydrazinoessigsäurederivaten der allgemeinen Formel III können bei der anschließenden Kondensation oder vor dieser als in sonstiger üblicher Weise abspaltbare übliche Schutzgruppen, für die X stehen kann, vorteilhaft die Trialkylsilyloxy-, Äthoxycarbonyloxy- und 2,4-Dinitrophenoxygruppe eingesetzt werden, wobei die Trimethylsilyloxygruppe und die Äthoxycarbonyloxygruppe besonders bevorzugt sind. Solche können auch in der Reaktionsmischung eingeführt werden.

Vorteilhaft können bei der Kondensation der Formylderivate von α-Hydrazinoessigsäurederivaten der allgemeinen Formel IV mit Aminopenicillansäure als solcher oder als Salz oder in Form von Estern bei der

Variante b) des erfindungsgemäßen Verfahrens Kondensationsmittel, insbesondere Dicyclohexylcarbodiimid, verwendet werden. Das gilt auch für die Formylierung.

Zweckmäßig wird die Kondensation der α-Hydrazinoessigsäurederivate der allgemeinen Formel V mit der 6-Aminopenicillansäure als solcher oder als Salz oder in Form von Estern bei der Variante c) des erfindungsgemäßen Verfahrens in aprotischen Lösungsmitteln und in Gegenwart von Halogenwasserstoffakzeptoren durchgeführt. Als erstere sind Polyhalogenkohlenwasserstoffe, wie Methylenchlorid und Chloroform, bevorzugt. Bevorzugte Halogenwasserstoffakzeptoren sind Propylenoxyd, Natriumbicarbonat und Dimethylanilin.

Das Entfernen der gegebenenfalls vorliegenden Schutzgruppen kann durch Hydrogenolyse oder Hydrolyse bewerkstelligt werden.

Die bei den Varianten b) und c) des erfindungsgemäßen Verfahrens gegebenenfalls eingesetzten Ester der 6-Aminopenicillansäure können vorteilhaft durch Verestern der 6-Aminopenicillansäure mit Kohlensäure-α-halogendialkylestern, wie Kohlensäure-α-chlordiäthylester, erhalten worden sein.

Die im erfindungsgemäßen Verfahren als Ausgangsstoffe verwendeten Verbindungen der allgemeinen Formeln II, III und V sind, soweit bei ihnen R für eine Phenylgruppe steht, aus der deutschen Offenlegungsschrift 23 55 740 und, soweit bei ihnen R für eine Thienylgruppe steht, aus Il Farmaco Scientifico, Band 31, Seite 549 bekannt.

Ferner sind erfindungsgemäß Arzneimittel, welche eine oder mehrere der erfindungsgemäßen Verbindungen als Wirkstoff beziehungsweise Wirkstoffe, gegebenenfalls zusammen mit einem oder mehreren üblichen pharmazeutischen Konfektionierungsmitteln, enthalten, vorgesehen.

Die erfindungsgemäßen Verbindungen haben nämlich, wie bereits erwähnt, wertvolle pharmakologische, insbesondere antibakterielle beziehungsweise baktericide Wirkungen. Ihre Wirkung zeigt sich sowohl gegen grampositive als auch gegen gramnegative Bakterien. Die Mindesthemmkonzentration von erfindungsgemäßen Verbindungen in vitro, ausgedrückt in γ/cm³, sind in der folgenden Tabelle zusammengestellt.

Tabelle

| Mikroorganismus | Verbindung<br>(R)-6ß-[α-(2'-Formyl-1'-methylhydrazino)-phenylacetamido]-penicillansäure<br>(Beispiel 10) | (R)-6ß-[α-(1',2'-Diformylhydrazino)-phenylacetamido]-penicillansäure<br>(Beispiel 9) | (R)-6ß-[α-(2'-Formylhydrazino)-phenylacetamido]-penicillansäure<br>(Beispiel 11) |
|---|---|---|---|
| Staphylococcus aureus | 0,19 | 0,19 | 0,048 |
| Staphylococcus aureus (Penicillinase erzeugender Stamm) | 3,12 | 6,25 | 1,56 |
| Streptococcus pyogenes | 0,048 | 0,097 | <0,012 |
| Streptococcus pneumoniae | 0,024 | 0,024 | 0,012 |

Tabelle (Fortsetzung)

| Mikroorganismus * | Verbindung (R)-6ß-[α-(2'-Formyl-1'-methylhydrazino)-phenylacetamido]-penicillansäure (Beispiel 10) | (R)-6ß-[α-(1',2'-Diformylhydrazino)-phenylacetamido]-penicillansäure (Beispiel 9) | (R)-6ß-[α-(2'-Formylhydrazino)-phenylacetamido]-penicillansäure (Beispiel 11) |
|---|---|---|---|
| Streptococcus faecalis | 3,12 | 6,25 | 1,56 |
| Sarcina lutea | 0,012 | 0,024 | < 0,012 |
| Escherichia coli | 1,56 | 3,12 | 0,78 |
| Shigella dysenteriae | 1,56 | 3,12 | 0,78 |
| Salmonella typhi | 3,12 | 6,25 | 1,56 |
| Salmonella typhymurium | 12,5 | 25 | 3,12 |
| Salmonella enteritidis | 25 | 25 | 6,25 |
| Neisseria meningitidis | 0,012 | 0,097 | < 0,012 |
| Pseudomonas aeruginosa | > 200 | > 200 | 200 |

Die Erfindung wird an Hand der folgenden Beispiele näher erläutert.

Beispiel 1

(R)-6β-[α-(2'-Formylhydrazino)-phenyl-acetamido]-penicillansäure-{pivaloyloxymethyl}-ester

Es wurden einer Lösung von 3 g R-( − )-α-[1-(p-Nitrobenzyloxycarbonyl)-hydrazino]-phenylessigsäure in 20 $cm^3$ Ameisensäure unter Rühren beziehungsweise Schütteln bei 4 °C 10 $cm^3$ des gemischten Anhydrides von Ameisensäure und Essigsäure während 10 Minuten zugetropft, worauf noch 1 Stunde bei Raumtemperatur gerührt beziehungsweise geschüttelt wurde. Das Reaktionsgemisch wurde zur Trockne eingedampft und der Rückstand wurde mehrere Male in Diäthyläther aufgenommen, bis eine feste Substanz erhalten wurde, welche unter Vakuum mit Diäthyläther zerrieben und dann in mehr Diäthyläther aufgenommen wurde. So wurden 3,06 g R-( − )-α-[1-(p-Nitrobenzyloxycarbonyl)-2-formylhydrazino]-phenylessigsäure mit einem Schmelzpunkt von 158 bis 161 °C (unter Zersetzung) und einem $[\alpha]_D$-Wert von − 82,47° (c = 1 in Tetrahydrofuran) erhalten.

Es wurde 0,78 g Dicyclohexylcarbodiimid zu einer Lösung von 1,20 g 6β-Aminopenicillansäurepiva-

loyloxymethylester in 5 $cm^3$ Tetrahydrofuran unter Rühren beziehungsweise Schütteln bei 4 °C zugegeben und dann wurden 1,4 g der wie vorstehend beschrieben erhaltenen R-( – )-α-[1-(p-Nitrobenzyloxycarbonyl)-2-formylhydrazino]-phenylessigsäure, welche mit 8 $cm^3$ Tetrahydrofuran verdünnt worden ist, zugetropft. Es wurde noch 10 Minuten bei 4 °C und dann 30 Minuten bei Raumtemperatur weitergerührt beziehungsweise- geschüttelt. Der unlösliche Rückstand wurde abfiltriert, zur Trockne eingedampft, in 30 $cm^3$ Methylenchlorid aufgenommen, je 2-mal mit je 10 $cm^3$ einer 5 %-igen Natriumbicarbonatlösung und je 10 $cm^3$ Wasser gewaschen, wasserfrei gemacht und zur Trockne eingedampft. Der Rückstand wurde mit Diisopropyläther zerrieben und durch Chromatographieren (Silicagel, 100 %-iger Diäthyläther) gereinigt. So wurden 1,93 g (R)-6β-{α-[1'- (p-Nitrobenzyloxycarbonyl)-2'-formylhydrazino -phenylacetamido]}-penicillansäure-pivaloyloxymethylester mit einem Schmelzpunkt von 76 bis 78,5 °C (unter Zersetzung) und einem $[\alpha]_D$-Wert von + 55,5° (c = 1 in Tetrahydrofuran) erhalten.

Es wurden zu einer Lösung von 1,2 g des wie vorstehend beschrieben erhaltenen (R)-6β-{α-[1'- (p-Nitrobenzyloxycarbonyl)-2'-formylhydrazino -phenylacetamido]}-penicillansäure-{pivaloyloxymethylesters} in einer Mischung aus 10 $cm^3$ 95 %-igem Äthylalkohol und 3 $cm^3$ Wasser unter Rühren beziehungsweise Schütteln 1,8 g von 5 Gew.-% Palladium auf Calciumcarbonat zugegeben. Dann wurde 20 Stunden lang Wasserstoff durchperlen gelassen. Das Reaktionsgemisch wurde über Kieselgur (Celite®) filtriert, zur Trockne eingedampft und mehrere Male in Äther aufgenommen, bis eine feste Substanz erhalten wurde. Diese wurde dann unter Vakuum in Diisopropyläther zerrieben und dann in mehr Diisopropyläther aufgenommen. Sie wurde durch Chromatographieren (Silicagel, Mischung von n-Hexan und Äthylacetat im Volumverhältnis von 1 : 1) gereinigt. So wurde 0,20 g (R)-6β-[α-(2'-Formylhydrazino)-phenylacetamido]-penicillansäurepivaloyloxymethylester mit einem Schmelzpunkt von 69,3 bis 74,8 °C (unter Zersetzung) erhalten.

## Beispiel 2

(R, S)-6β-[α-(1'-Formylhydrazino)-phenylacetamido]-penicillansäure

Es wurde eine Lösung von 1,86 $cm^3$ Ameisensäure in 7,5 $cm^3$ Diäthyläther unter Kühlen auf – 15 °C mit einer Lösung von 3,43 g Dicyclohexylcarbodiimid in 10 $cm^3$ Diäthyläther versetzt und 30 Minuten lang gerührt beziehungsweise geschüttelt. Zur so erhaltenen Mischung wurde eine Suspension von (R, S)-α-[2-(Benzyloxycarbonyl)-hydrazino]-phenylessigsäuretrimethylsilylester, welcher durch Erhitzen der freien Säure (R, S)-α-[-(Benzyloxycarbonyl)-hydrazino]-phenylessigsäure mit Hexamethyldisilazan in Acetonitril unter Rückfluß zum Sieden erhalten worden ist, in Methylenchlorid bei 0 °C zugegeben und es wurde 2 Stunden lang bei 0 °C gerührt beziehungsweise geschüttelt. Dann wurden 50 $cm^3$ kaltes Wasser zugegeben und der pH-Wert wurde auf 9 eingestellt. Der feste Rückstand wurde abfiltriert und die Schichten wurden voneinander getrennt. Die wäßrige Schicht wurde auf den pH-Wert von 1,5 angesäuert und 3-mal mit je 50 $cm^3$ Methylenchlorid extrahiert und die organischen Auszüge wurden vereinigt und mit 20 $cm^3$ Wasser gewaschen, wasserfrei gemacht und zur Trockne eingedampft. So wurden 2,64 g (R, S)-α-[1'-Formyl-2'-(benzyloxycarbonyl)-hydrazino]-phenylessigsäure als Öl erhalten, aus welchem das entsprechende Natriumsalz mit einem Schmelzpunkt von 148 bis 150 °C (unter Zersetzung) erhalten wurde. Zu einer Lösung von 10 g dieses Natriumsalzes von (R, S-α-[1'-Formyl-2'-(benzyloxycarbonyl)-hydrazino]-phenylessigsäure in 85 $cm^3$ Aceton wurden unter Kühlen auf – 40 °C 2,95 $cm^3$ Chlorameisensäureäthylester zugegeben und dann wurde die Mischung 30 Minuten lang gerührt beziehungsweise geschüttelt. Danach wurden 30 $cm^3$ einer wäßrig-acetonischen Lösung von 5,85 g des Triäthylaminsalzes von 6β-Aminopenicillansäure zugegeben und die Mischung wurde 45 Minuten lang bei – 25 °C gerührt beziehungsweise geschüttelt. Daraufhin wurden der Mischung 50 $cm^3$ kaltes Wasser und 150 $cm^3$ Methylenchlorid zugesetzt und sie wurde auf den pH-Wert von 2 angesäuert. Dann wurden die Schichten voneinander getrennt und die organische Schicht wurde mit 50 $cm^3$ Wasser gewaschen, wasserfrei gemacht und zur Trockne eingedampft. Der Rückstand wurde mit Cyclohexan zerrieben. So wurden 13 g (R, S)-6β-{α-[1'-Formyl-2'-(benzyloxycarbonyl)-hydrazino]}-phenylacetamidopenicillansäure mit einem Schmelzpunkt von 98 bis 100 °C (unter Zersetzung) erhalten.

Es wurden zu einer Lösung von 1,16 g Natriumbicarbonat in 37,5 $cm^3$ Wasser 7,3 g der wie vorstehend beschrieben erhaltenen (R, S)-6β-{α-[1'-Formyl-2'-(benzyloxycarbonyl)-hydrazino]}-phenylacetamidopenicillansäure und 5,25 g von 10 Gew.-% Palladium auf Holzkohle zugegeben. Es wurde 2 Stunden lang Wasserstoff durchperlen gelassen, dann wurde der Katalysator abfiltriert und das Filtrat mit 130 $cm^3$ Methylenchlorid überschichtet, auf 0 °C gekühlt und auf den pH-Wert von 2 angesäuert. Die Schichten wurden voneinander getrennt, die organische Schicht wurde 2-mal mit je 20 $cm^3$ Methylenchlorid extrahiert und die organischen Auszüge wurden vereinigt und 3-mal mit je 30 $cm^3$ Wasser gewaschen, wasserfrei gemacht und zur Trockne eingedampft. So wurden 2,15 g (R, S)-6β-[α-1'-Formylhydrazino)-phenylacetamido]-penicillansäure mit einem Schmelzpunkt von 156 bis 160 °C (unter Zersetzung) erhalten.

## Beispiel 3

(R)-6β-[α-2'Formylhydrazino)-phenylacetamido]-penicillansäure

Es wurden zu einer Lösung von 2,26 g 6β-Aminopenicillansäure-p-nitrobenzylester in 10 $cm^3$ Tetrahydrofuran unter Rühren beziehungsweise Schütteln bei 4 °C 1,33 g Dicyclohexylcarbodiimid und dann 2,40 g R-( – )-α-[1-(p-Nitrobenzyloxycarbonyl)-2-formylhydrazino]-phenylessigsäure in Lösung in 20 $cm^3$ Tetrahydrofuran, welche wie im Beispiel 1 beschrieben erhalten worden ist, zugegeben. Die Mischung wurde noch 10 Minuten bei 4 °C und 3 Stunden bei Raumtemperatur weitergerührt beziehungsweise -geschüttelt, dann wurde der Rückstand durch Filtrieren entfernt und das Filtrat wurde zur Trockne eingedampft und mehrere Male in Diäthyläther aufgenommen, bis eine feste Substanz erhalten wurde. Diese wurde in 50 $cm^3$ Äthylacetat gelöst, 3-mal mit je 10 $cm^3$ einer 5 %-igen Natriumbicarbonatlösung gewaschen, wasserfrei gemacht und zur Trockne eingedampft. Sie wurde durch Chromatographieren (Silicagel, Mischung aus n-Hexan und Äthylacetat im Volumverhältnis von 3 : 7) gereinigt. So wurden 3,42 g (R)-6β-{α-[1'-(p-Nitrobenzyloxycarbonyl)-2'-formylhydrazino]}-phenylacetamidopenicillansäure-p-nitrobenzylester mit einem Schmelzpunkt von 101,5 bis 104,7 °C (unter Zersetzung) und einem $[\alpha]_D$-Wert von + 74,47° (c = 1 in Tetrahydrofuran) erhalten.

Es wurden zu einer Lösung von 2,20 g des wie vorstehend beschrieben erhaltenen (R)-6β-{α-[1'-(p-Nitrobenzyloxycarbonyl)-2'-formylhydrazino]}-phenylacetamidopenicillansäure-p-nitrobenzylesters in einer Mischung aus 30 $cm^3$ Tetrahydrofuran und 14 $cm^3$ Wasser unter Rühren beziehungsweise Schütteln 4,50 g von 5 Gew.-% Palladium auf Calciumcarbonat zugegeben. Es wurde 20 Stunden lang Wasserstoff durchperlen gelassen, über Kieselgur filtriert und zur Trockne eingedampft. Der Rückstand wurde mit Äther verrieben, durch Filtrieren unter Vakuum gesammelt und in einer Mischung aus 5,5 $cm^3$ Tetrahydrofuran und 1 $cm^3$ Wasser gelöst. Zur Lösung wurden unter Rühren beziehungsweise Schütteln bei 4 °C 50 $cm^3$ Isopropylalkohol zugegeben und es wurde noch 10 Minuten weitergerührt beziehungsweise-geschüttelt und dann wurde der feste Rückstand durch Filtrieren unter Vakuum gesammelt. So wurden 1,09 g (R)-6β-[α-(2'-Formylhydrazino)-phenylacetamido]-penicillansäure als Calciumsalz mit einem Schmelzpunkt von 200 °C (unter Zersetzung) erhalten.

## Beispiel 4

(R)-6β-[α-(2'-Formylhydrazino)-phenyl-acetamido]-penicillansäure-{1''-(äthoxycarbonyloxy)-äthyl}-ester

Es wurde zu einer Lösung von 1,21 g 6β-Aminopenicillansäure-[1'-(äthoxycarbonyloxy)-äthyl]-ester in 5 $cm^3$ Tetrahydrofuran unter Kühlen auf 4 °C 0.78 g Dicyclohexylcarbodiimid zugegeben und dann wurde eine Lösung von 1,40 g R-( – )-α-[1-p-Nitrobenzyloxycarbonyl)-2-formylhydrazino]-phenylessigsäure, welche wie im Beispiel 1 beschrieben erhalten worden ist, zugetropft. Es wurde 30 Minuten lang bei Raumtemperatur gerührt beziehungsweise geschüttelt. Die feste Substanz wurde abfiltriert und das Filtrat wurde zur Trockne eingedampft. Der Rückstand wurde in 30 $cm^3$ Methylenchlorid gelöst, je 2-mal mit je 10 $cm^3$ einer 5 %-igen Natriumbicarbonatlösung und je 10 $cm^3$ Wasser gewaschen, dann wasserfrei gemacht und zur Trockne eingedampft. Der Rückstand wurde durch Chromatographieren (Silicagel, Äther) gereinigt. So wurden 1,6 g (R)-6β-{α-[1'-(p-Nitrobenzyloxycarbonyl)-2'-formylhydrazino]}-phenylacetamidopenicillansäure-{1''-(äthoxycarbonyloxy)äthyl}-ester mit einem Schmelzpunkt von 89,3 bis 97,3 °C erhalten.

Es wurden zu einer Lösung des wie vorstehend beschrieben erhaltenen (R)-6β-{α-[1'-(p-Nitrobenzyloxycarbonyl)-2'-formylhydrazino]}-phenylacetamidopenicillansäure-{1''-(äthoxycarbonyloxy)-äthyl}-esters in einem Gemisch von 10 $cm^3$ 95 %-igem Äthylalkohol und 3 $cm^3$ Wasser 1,80 g von 5 Gew.-% Palladium auf Calciumcarbonat zugegeben und 20 Stunden lang Wasserstoff durchperlen gelassen. Der Katalysator wurde abfiltriert, die Mischung wurde zur Trockne eingedampft und dann wurde der Rückstand durch Chromatographieren (Silicagel, Mischung von n-Hexan und Äthylacetat im Volumverhältnis von 1 : 1) gereinigt. So wurde 0,43 g (R)-6β-[α-(2'-Formylhydrazino)-phenylacetamido]-penicillansäure-{1''-(äthoxycarbonyloxy)-äthyl}-ester mit einem Schmelzpunkt von 58,6 bis 64,3 °C und einem $[\alpha]_D$-Wert von + 111,3° (c = 1 in Chloroform) erhalten.

## Beispiel 5

(R)-6β-[α-(1',2'-Diformylhydrazino)-phenyl-acetamido]-penicillansäurepivaloyloxymethylester

Es wurden 3 $cm^3$ des gemischten Anhydrides von Essigsäure und Ameisensäure einer Lösung von 1 g R-( – )-α-Hydrazinophenylessigsäure in 3 $cm^3$ Ameisensäure unter Rühren beziehungsweise Schütteln bei 4 °C zugetropft und das Rühren beziehungsweise Schütteln wurde noch 10 Minuten bei dieser Temperatur und dann noch 30 Minuten bei Raumtemperatur fortgesetzt. Das Reaktionsgemisch wurde zur Trockne eingedampft und in Diäthyläther aufgenommen und die erhaltene feste Substanz wurde durch Filtrieren unter Vakuum gesammelt. So wurde 0,94 g R-( – )-α-(1,2-Diformylhydrazino)-phenylessigsäure mit einem Schmelzpunkt von 141,7 bis 155,4 °C (unter Zersetzung) und einem $[\alpha]_D$-Wert von – 222,5 ° (c = 1 in Tetrahydrofuran) erhalten.

Es wurden einer Lösung von 2,97 g 6β-Aminopenicillansäurepivaloyloxymethylester in 10 $cm^3$ Tetrahydrofuran unter Rühren beziehungsweise Schütteln bei 4 °C 1,85 g Dicyclohexylcarbodiimid zugesetzt und eine Lösung von 2 g wie vorstehend beschrieben erhaltener R( – )-α-(1,2-Diformylhydrazi-

no)-phenylessigsäure in 30 $cm^3$ Tetrahydrofuran zugetropft. Es wurde noch 10 Minuten bei 4 °C und dann noch 2 Stunden bei Raumtemperatur weitergerührt beziehungsweise-geschüttelt. Dann wurde filtriert, das Filtrat wurde zur Trockne eingedampft und der Rückstand wurde mit Diisopropyläther zerrieben, durch Filtrieren unter Vakuum gesammelt und chromatographiert (Silicagel, Mischung aus n-Hexan und Äthylacetat im Volumverhältnis von 3 : 7). So wurden 2,4 g (R)-6β-[α-(1',2'-Diformylhydrazino)-phenylacetamido]-penicillansäurepivaloyloxymethylester mit einem Schmelzpunkt von 91,7 bis 93,1 °C und einem $[\alpha]_D$-Wert von + 96,42° (c = 1 in Tetrahydrofuran) erhalten.

## Beispiel 6

(R, S)-6β-[α-(1',2'-Diformylhydrazino)-thien-2''-ylacetamido]-penicillansäurepivaloyloxymethylester

Es wurde einer Lösung von 1 g (R, S)-α-Hydrazinothien-2-ylessigsäure in 3 $cm^3$ Ameisensäure unter Rühren beziehungsweise Schütteln bei 4 °C eine Lösung von 3 $cm^3$ des gemischten Anhydrides von Essigsäure und Ameisensäure zugetropft. Das Rühren beziehungsweise Schütteln wurde noch 20 Minuten bei derselben Temperatur und noch 30 Minuten bei Raumtemperatur fortgesetzt. Dann wurde die Reaktionsmischung zur Trockne eingedampft und in Diäthyläther aufgenommen und die so erhaltene feste Substanz wurde mit Äthyläther zerrieben und durch Filtrieren unter Vakuum gesammelt. So wurden 1,26 g (R, S)-α-(1,2-Diformylhydrazino)-thien-2'-ylessigsäure mit einem Schmelzpunkt von 168 bis 170 °C (unter Zersetzung) erhalten.

Es wurden einer Lösung von 1,1 g 6β-Aminopenicillansäurepivaloyloxymethylester in 3 $cm^3$ Tetrahydrofuran unter Rühren beziehungsweise Schütteln bei Raumtemperatur 686 mg Dicyclohexylcarbodiimid zugesetzt und eine Lösung von 760 mg der wie vorstehend beschrieben erhaltenen (R, S)-α-(1,2-Diformylhydrazino)-thien-2'-ylessigsäure in 20 $cm^3$ Tetrahydrofuran zugetropft. Die Mischung wurde 1 1/2 Stunden lang gerührt beziehungsweise geschüttelt, der Rückstand wurde durch Filtrieren entfernt, das Filtrat wurde zur Trockne eingedampft und der Rückstand wurde mit Diisopropyläther zerrieben, durch Filtrieren unter Vakuum gesammelt und durch Chromatographieren (Silicagel, Mischung aus n-Hexan und Äthylacetat im Volumverhältnis von 3 : 7) gereinigt. So wurden 1,25 g (69 % der Theorie) (R, S)-6β-[α-(1',2'-Diformylhydrazino)-thien-2''-ylacetamido]-penicillansäurepivaloyloxymethylester mit einem Schmelzpunkt von 67,4 °C und einem $[\alpha]_D$-Wert von + 147,2 ° (c = 1 in Tetrahydrofuran) erhalten.

## Beispiel 7

(R)-6β-[α-(1'-Methyl-2'-formylhydrazino)-phenylacetamido]-penicillansäurepivaloyloxymethylester

Es wurden einer Lösung von 5 g (R)-α-(1-Methylhydrazino)-phenylessigsäure in 10 $cm^3$ Ameisensäure unter Rühren beziehungsweise Schütteln bei 4 °C 15 $cm^3$ des gemischten Anhydrides von Essigsäure und Ameisensäure zugetropft und die Mischung wurde noch 15 Minuten bei 4 °C und dann noch 1 1/2 Stunden bei Raumtemperatur gerührt beziehungsweise geschüttelt. Sie wurde zur Trockne eingedampft und der ölige Rückstand wurde in eine Mischung von 30 $cm^3$ Methylenchlorid und 30 $cm^3$ Wasser aufgenommen und ihr pH-Wert wurde auf 7,2 eingestellt und die wäßrige Schicht wurde mit Methylenchlorid gewaschen und mit 50 $cm^3$ Methylenchlorid überschichtet, ihr pH-Wert wurde auf 2 eingestellt und sie wurde mit Methylenchlorid extrahiert. Die organischen Schichten wurden vereinigt, wasserfrei gemacht, zur Trockne eingedampft und mit Diäthyläther zerrieben, wodurch ein fester Rückstand erhalten wurde, welcher durch Filtrieren unter Vakuum gesammelt wurde. So wurden 3,46 g (R)-α-(2-Formyl-1-methylhydrazino)-phenylessigsäure mit einem Schmelzpunkt von 129 bis 131 °C und einem $[\alpha]_D$-Wert von 104,8° (c = 1 in einer 0,1 n Natriumhydroxydlösung) erhalten.

Es wurde zu einer Lösung von 0,5 g der wie vorstehend beschrieben erhaltenen (R)-α-(2-Formyl-1-methylhydrazino)-phenylessigsäure und 0,34 $cm^3$ Triäthylamin in 5 $cm^3$ Tetrahydrofuran bei − 50 °C 0,25 $cm^3$ Chlorameisensäureäthylester zugegeben, die Mischung wurde 30 Minuten lang bei − 20 °C gerührt beziehungsweise geschüttelt und dann wurde eine Lösung von 0,72 g 6β-Aminopenicillansäurepivaloyloxymethylester in 8 $cm^3$ Tetrahydrofuran zugesetzt. Die Mischung wurde 1 Stunde lang bei 0 °C gerührt beziehungsweise geschüttelt, worauf eine Mischung von 20 $cm^3$ Methylenchlorid und 20 $cm^3$ Wasser zugesetzt wurde. Die Schichten wurden voneinander getrennt und die organische Schicht wurde je 2-mal mit je 10 $cm^3$ einer 5 %-igen Natriumbicarbonatlösung und je 10 $cm^3$ Wasser gewaschen. Sie wurde wasserfrei gemacht und zur Trockne eingedampft. Der Rückstand wurde durch Chromatographieren (Silicagel, Mischung von n-Hexan und Äthylacetat im Volumverhältnis von 10 : 2) gereinigt. So wurde 0,30 g (R)-6β-[α-(1'-Methyl-2'-formylhydrazino)-phenylacetamido]-penicillansäurepivaloyloxymethylester mit einem Schmelzpunkt von 147 bis 154,3 °C (unter Zersetzung) und einem $[\alpha]_D$-Wert von + 129,4° (c = 0,5 in Chloroform) erhalten.

## Beispiel 8

(R)-6β-[α-(1'-Methyl-2'-formylhydrazino)-phenylacetamido]-penicillansäure-{1''-(äthoxycarbonyloxy)-äthyl}-ester

Es wurde wie im Beispiel 7 beschrieben gearbeitet, jedoch der 6β-Aminopenicillansäure-{1'-(äthoxycarbonyloxy)-äthyl}-ester an Stelle des 6β-Aminopenicillansäurepivaloyloxymethylesters als Ausgangsverbindung eingesetzt. So wurde (R)-6β-[α-(1'-Methyl-2'-formylhydrazino)-phenylacetamido]-penicillansäure-{1''-(äthoxycarbonyloxy)-äthyl}-ester mit einem Schmelzpunkt von 71,3 bis 77,4 °C (unter Zersetzung) und einem $[\alpha]_D$-Wert von 131,6° (c = 0,5 in Chloroform) erhalten.

Beispiel 9

(R)-6β-[α-(1',2'-Diformylhydrazino)-phenylacetamido]-penicillansäure

Es wurden einer Lösung von 0,5 g (R)-6β-(α-Hydrazinophenylacetamido)-penicillansäure in 6 $cm^3$ Pyridin unter Rühren beziehungsweise Schütteln bei 4 °C 1,5 $cm^3$ des gemischten Anhydrides von Essigssäure und Ameisensäure zugetropft und es wurde noch 5 Stunden weitergerührt beziehungsweise-geschüttelt. Dann wurde 3-mal mit 10 $cm^3$ n-Hexan gewaschen und der ölige Rückstand wurde mit 30 $cm^3$ Diäthylätherzerrieben und durch Filtrieren unter Vakuum gesammelt. So wurde 0,38 g (R)-6β-[α-(1',2'-Diformylhydrazino)-phenylacetamido]-penicillansäure mit einem Schmelzpunkt von 137 bis 177 °C (unter Zersetzung) erhalten. Ihr durch Behandlung mit einer Lösung des Natriumsalzes der 2-Äthylcapronsäure in Isopropylalkohol erhaltenes Natriumsalz hatte einen Schmelzpunkt von 180 °C (unter Zersetzung).

Beispiel 10

(R)-6β-[α-(1'-Methyl-2'-formyl-hydrazino)-phenylacetamido]-penicillansäure

Es wurden einer Lösung von 2 g (R)-6β-[α-(1'-Methylhydrazino)-phenylacetamido]-penicillansäure in 15 $cm^3$ Pyridin unter Rühren beziehungsweise Schütteln bei 4 °C 6 $cm^3$ des gemischten Anhydrides von Essigsäure und Ameisensäure zugetropft. Das Ganze wurde noch 30 Minuten bei derselben Temperatur weitergerührt beziehungsweise-geschüttelt. Die Mischung wurde mit n-Hexan gewaschen, bis ein dickes öliges Produkt erhalten wurde, welches 3-mal mit je 50 $cm^3$ Diäthyläther zerrieben wurde. Unter Vakuum wurden 1,5 g (R)-6β-[α-(1'-Methyl-2'-formyl-hydrazino)-phenylacetamido]-penicillansäure mit einem Schmelzpunkt von 157 bis 158,1 °C (unter Zersetzung) und einem $[\alpha]_D$-Wert von + 201,51° (c = 1 in Tetrahydrofuran) abgetrennt.

Beispiel 11

(R)-6β-[α-(2'-Formylhydrazino)-phenylacetamido]-penicillansäure

Es wurden einer Lösung von 5 g (R)-6β-(α-Hydrazinophenylacetamido)-penicillansäure und 5,4 $cm^3$ Triäthylamin in 100 $cm^3$ Methylenchlorid unter Kühlen auf – 20 °C 2,3 $cm^3$ des gemischten Anhydrides von Essigsäure und Ameisensäure in 20 $cm^3$ Methylenchlorid zugetropft. Die Mischung wurde 30 Minuten lang bei – 20 °C und dann 1 Stunde lang bei Raumtemperatur gerührt beziehungsweise geschüttelt. Sie wurde zur Trockne eingedampft und der Rückstand wurde mit 80 $cm^3$ einer 3,5 %-igen Natriumbicarbonatlösung verdünnt. Es wurde mit 40 $cm^3$ Äthylacetat überschichtet und die Schichten wurden voneinander getrennt. Die wäßrige Schicht wurde mit 100 $cm^3$ Äthylacetat erneut überschichtet und dann mit einer 10 %-igen Salzsäure auf einen pH-Wert von 2 eingestellt. Die Schichten wurden voneinander getrennt und die organische Schicht wurde wasserfrei gemacht und eingeengt, der feste Rückstand wurde durch Filtrieren entfernt und das klare Filtrat wurde zur Trockne eingedampft. Der Rückstand wurde mit Diäthyläther zerrieben. So wurden 2,6 g (R)-6β-[α-(2'-Formylhydrazino)-phenylacetamido]-penicillansäure mit einem Schmelzpunkt von 145,5 bis 147,8 °C (unter Zersetzung) und einem $[\alpha]_D$-Wert von + 133,8° (c = 1 in Tetrahydrofuran) erhalten.

Beispiel 12

(R)-6β-[α-(1'-Methyl-2'-formylhydrazino)-phenylacetamido]-penicillansäurepivaloyloxymethylester

Es wurden zu einer Lösung von 3,3 g 6β-Aminopenicillansäurepivaloyloxymethylester in 30 $cm^3$ Methylenchlorid unter Rühren beziehungsweise Schütteln und unter Kühlen auf 0 °C 13 $cm^3$ Propylenoxyd und dann unter Kühlen auf –30 °C 2,3 g (R)-α-(1-Methylhydrazino)-phenylacetylchloridhydrochlorid zugegeben. Es wurde noch 30 Minuten bei – 20 °C und noch 2 Stunden bei 0 °C gerührt beziehungsweise geschüttelt. Dann wurde zur Trockne eingedampft und der Rückstand wurde mit Diisopropyläther zerrieben. So wurden 4,2 g (R)-6β-[α-(1'-Methylhydrazino)-phenylacetamido]-penicillansäurepivaloyloxymethylesterhydrochlorid mit einem Schmelzpunkt von 88 bis 90 °C (unter Zersetzung) und einem $[\alpha]_D$-Wert von + 140,53° (c = 1 in Methylalkohol) erhalten.

Es wurden einer Lösung von 4 g des wie vorstehend beschrieben erhaltenen (R)-6β-[α-(1'-Methylhydrazino)-phenylacetamido]-penicillansäurepivaloyloxymethylesterhydrochlorides in 25 $cm^3$

wasserfreiem Pyridin unter Kühlen auf 4 °C 4,2 $cm^3$ des gemischten Anhydrides von Essigsäure und Ameisensäure zugetropft. Es wurde 30 Minuten lang bei 4 °C weitergerührt beziehungsweise-geschüttelt, dann zur Trockne eingedampft und der Rückstand wurde in 100 $cm^3$ Äthylacetat gelöst und mit Wasser gewaschen. Die organische Schicht wurde wasserfrei gemacht und zur Trockne eingedampft und dann wurde der Rückstand durch Chromatographieren (Silicagel, Mischung von n-Hexan und Äthylacetat im Volumverhältnis von 10 : 2) gereinigt. So wurden 2,50 g (R)-6β-[α-(1'-Methyl-2'-formylhydrazino)-phenylacetamido]-penicillansäurepivaloyloxymethylester mit einem Schmelzpunkt von 147 bis 154,3 °C (unter Zersetzung) und einem $[\alpha]_D$-Wert von + 129,4° (c = 0,5 in Chloroform) erhalten.

## Beispiel 13

(R)-6β-[α-(1'-Methyl-2'-formylhydrazino)-phenylacetamido]-penicillansäure-{1''-(äthoxycarbonyloxy)-äthyl}-ester

Es wurden 24 $cm^3$ Propylenoxyd zu einer Lösung von 6 g 6β-Aminopenicillansäure-{1'-(äthoxycarbonyloxy)-äthyl}-ester in 60 $cm^3$ Methylenchlorid zugegeben. Die Mischung wurde auf – 30 °C gekühlt und unter Rühren beziehungsweise Schütteln wurden 4,25 g (R)-α-(1-Methylhydrazino)-phenyl-acetylchloridhydrochlorid zugesetzt. Die Temperatur wurde 30 Minuten lang auf – 30 °C gehalten, dann auf Raumtemperatur erhöht und es wurde noch 1 Stunde bei der letzteren weitergerührt beziehungsweise-geschüttelt. Die Mischung wurde zur Trockne eingedampft und der Rückstand wurde mit Diäthyläther zerrieben und mit Natriumbicarbonat behandelt. So wurden 7 g (R)-6β-[α-(1'-Methylhydrazino)-phenylacetamido]-penicillansäure-{1''-(äthoxycarbonyloxy)-äthyl}-ester mit einem Schmelzpunkt von 45,6 bis 47 °C (unter Zersetzung) und einem $[\alpha]_D$-Wert von + 76,7 ° (c = 0,5 in Chloroform) erhalten.

Es wurden 10 $cm^3$ des gemischten Anhydrides von Essigsäure und Ameisensäure einer Lösung 5 g (R)-6β-[α-(1'-Methylhydrazino)-phenylacetamido]-penicillansäure-{1''-(äthoxycarbonyloxy)-äthyl}-ester in 40 $cm^3$ wasserfreiem Pyridin unter Kühlen auf 4 °C und unter Rühren beziehungsweise Schütteln während 30 Minuten zugetropft. Zu dieser Mischung wurden 150 $cm^3$ Äthylacetat zugegeben, sie wurde 4-mal mit je 100 $cm^3$ Wasser gewaschen und die organische Schicht wurde nach ihrer Abtrennung wasserfrei gemacht und zur Trockne eingedampft. Der Rückstand wurde durch Chromatographieren (Silicagel, Mischung von Äthylacetat und n-Hexan im Volumverhältnis von 9 : 1) gereinigt. So wurden 2,20 g (R)-6β-[α-(1'-Methyl-2'-formylhydrazino)-phenylacetamido]-penicillansäure-{1''-(äthoxycarbonyloxy)-äthyl}-ester mit einem Schmelzpunkt von 71,3 bis 77,4 °C (unter Zersetzung) und einem $[\alpha]_D$-Wert von + 131,6° (c = 0,5 in Chloroform) erhalten.

## Beispiel 14

(R)-6β-[α-(1'-Methyl-2'-formylhydrazino)-phenylacetamido]-penicillansäure-{1''-(äthoxycarbonyloxy)-äthyl}-ester

Es wurden zu einer Lösung von 0,80 g wie im Beispiel 10 beschrieben erhaltenem (R)-6β-[α-(1'-Methyl-2'-formylhydrazino)-phenylacetamido]-penicillansäure in 5 $cm^3$ Dimethylsulfoxyd 0,28 $cm^3$ Triäthylamin und 0,30 g Kohlensäure-α-chlordiäthylester zugegeben. Die Mischung wurde 16 Stunden lang gerührt beziehungsweise geschüttelt und dann wurden 20 $cm^3$ Äthylacetat zugesetzt. Sie wurde 4-mal mit je 20 $cm^3$ Wasser gewaschen und die organische Schicht wurde abgetrennt und danach wasserfrei gemacht und zur Trockne eingedampft. Der Rückstand wurde aus einer Mischung aus Isopropylalkohol und Wasser umkristallisiert. So wurde 0,15 g (R)-6β-[α-(1'-Methyl-2'-formylhydrazino)-phenylacetamido]-penicillansäure-{1''-(äthoxycarbonyloxy)-äthyl}-ester, welcher der nach dem Beispiel 13 erhaltenen Verbindung gleich war, erhalten.

## Beispiel 15

(R)-6β-[α-(2'-Formylhydrazino)-phenylacetamido]-penicillansäure-{1''-(äthoxycarbonyloxy)-äthyl}-ester

Es wurden zu einer Lösung von 13,28 g 6β-Aminopenicillansäure-{1'-(äthoxycarbonyloxy)-äthyl}-ester in 150 $cm^3$ Methylenchlorid 40 $cm^3$ Propylenoxyd zugegeben. Die Reaktionsmischung wurde auf – 30 °C gekühlt, zu ihr wurden 8,85 g (R)-α-Hydrazinophenylacetylchloridhydrochlorid zugegeben und sie wurde 1 Stunde lang unter Rühren beziehungsweise Schütteln auf derselben Temperatur gehalten. Durch Eindampfen zur Trockne und Zerreiben des öligen Rückstandes mit Diäthyläther wurden 20 g (R)-6β-(α-Hydrazinophenylacetamido)-penicillansäure-{1''-(äthoxycarbonyloxy)-äthyl}-esterhydrochlorid mit einem Schmelzpunkt von 108,4 bis 109,6 °C und einem $[\alpha]_D$-Wert von + 109,9° (c = 1 in Chloroform) erhalten.

Es wurden zu einer Lösung von 15 g des wie vorstehend beschrieben erhaltenen (R)-6β-(α-Hydrazinophenylacetamido)-penicillansäure-{1''-(äthoxycarbonyloxy-äthyl}-esterhydrochlorides in 150 $cm^3$ Pyridin unter Kühlen auf 0 °C langsam 4,95 $cm^3$ des gesmischten Anhydrides von Essigsäure und Ameisensäure in 30 $cm^3$ Chloroform zugegeben. Die Mischung wurde 30 Minuten lang bei 0 °C und noch

1 Stunde bei Raumtemperatur gerührt beziehungsweise geschüttelt und dann wurden 500 $cm^3$ Äthylacetat zugesetzt und es wurde 4-mal mit je 100 $cm^3$ Wasser gewaschen. Die organische Schicht wurde wasserfrei gemacht und zur Trockne eingedampft, der Rückstand wurde in Petroläther aufgenommen bis ein gummiartiges Produkt erhalten wurde, welches dann durch Chromatographieren (Silicagel, Mischung aus n-Hexan und Äthylacetat im Volumverhältnis von 1 : 1) gereinigt wurde. So wurden 2,35 g (R)-6β-[α-(2'-Formylhydrazino)-phenylacetamido]-penicillansäure-{1"-(äthoxycarbonyloxy)-äthyl}-ester mit einem Schmelzpunkt von 58,6 bis 64,3 °C und einem $[\alpha]_D$-Wert von + 111,3° (c = 1 in Chloroform) erhalten.

## Beispiel 16

(R)-6β-[α-(2'-Formylhydrazino)-phenylacetamido]-penicillansäurepivaloyloxymethylester

Es wurde wie im Beispiel 15 beschrieben gearbeitet, jedoch als Ausgangsmaterial 6β-Aminopenicillansäurepivaloyl-oxymethylester an Stelle des 6β-Aminopenicillansäure-{1'-(äthoxycarbonyloxy)-äthyl}-esters eingesetzt. So wurde zunächst (R)-6β-(α-Hydrazinophenylacetamido)-penicillansäurepivaloyloxymethylesterhydrochlorid und dann (R)-6β-[α-(2'-Formylhydrazino)-phenylacetamido]-penicillansäurepivaloyloxymethylester mit einem Schmelzpunkt von 69,3 bis 74,8 °C (unter Zersetzung) erhalten.

## Beispiel 17

(R)-6β-[α-(2'-Formylhydrazino)-phenylacetamido]-penicillansäure

Es wurde zu einer Suspension von 6,57 g wie im Beispiel 1 beschrieben hergestellter R( − )-α-[1-(p-Nitrobenzyloxycarbonyl)-2-formylhydrazino]-phenylessigsäure in 60 $cm^3$ Wasser unter Rühren beziehungsweise Schütteln eine Lösung von 0,95 g Natriumcarbonat zugegeben. Der so erhaltenen Mischung wurden 10 g von 5 Gew.-% Palladium auf Calciumcarbonat zugesetzt und es wurde 13 Stunden lang Wasserstoff durchperlen gelassen. Die Reaktionsmischung wurde über Kieselgur filtriert, mit Chloroform gewaschen, ihr pH-Wert wurde auf 2 eingestellt und sie wurde eingeengt, bis ein Niederschlag erhalten wurde, welcher unter Vakuum gesammelt wurde. So wurden 1,39 g R-( − )-α-(2-Formylhydrazino)-phenylessigsäure erhalten. Die wäßrigen Mutterlaugen wurden nach ihrem Eindampfen zur Trockne mit Tetrahydrofuran extrahiert. Die organische Schicht wurde nach dem Entfernen des Lösungsmittels mit Diathyläther zerrieben. Die feste Substanz wurde durch Filtrieren unter Vakuum gesammelt. So wurde 0,85 g R-( − )-α-(2-Formylhydrazino)-phenylessigsäure mit einem Schmelzpunkt von 136,9 °C und einem $[\alpha]_D$-Wert von 169° (c = 1 in Tetrahydrofuran) erhalten.

Es wurden einer Lösung von 3,4 g 6β-Aminopenicillansäure-p-nitrobenzylester in 20 $cm^3$ Tetrahydrofuran unter Rühren beziehungsweise Schütteln bei 4 °C 2 g Dicyclohexylcarbodiimid zugesetzt und eine Lösung von 1,88 g wie vorstehend beschrieben erhaltener R-( − )-α-(2-Formylhydrazino)-phenylessigsäure in 30 $cm^3$ Tetrahydrofuran zugetropft. Das Rühren beziehungsweise Schütteln wurde 1 Stunde lang bei 4 °C und dann 5 Stunden lang bei Raumtemperatur fortgesetzt. Das unlösliche Material wurde abfiltriert und das Filtrat wurde zur Trockne eingedampft und durch Chromatographieren (Silicagel, Mischung aus n-Hexan und Äthylacetat im Volumverhältnis von 3 : 7) gereinigt. So wurden 1,72 g (R)-6β-[α-(2'-Formylhydrazino)-phenylacetamido]-penicillansäure-p-nitrobenzylester mit einem Schmelzpunkt von 84,9 bis 86,9 °C und einem $[\alpha]_D$-Wert von + 127° (c = 1 in Tetrahydrofuran) erhalten.

Es wurde zu einer Lösung von 0,5 g des wie vorstehend beschrieben erhaltenen (R)-6β-[α-(2'-Formylhydrazino)-phenylacetamido]-penicillansäure-p-nitrobenzylesters in einer Mischung von 8 $cm^3$ Tetrahydrofuran und 4 $cm^3$ Wasser 0,35 g von 10 Gew.-% Palladium auf Holzkohle unter Rühren beziehungsweise Schütteln zugegeben und es wurde 7 Stunden lang Wasserstoff durchperlen gelassen. Es wurde über Kieselgur filtriert und mit Tetrahydrofuran und einer 5 %-igen wäßrigen Natriumbicarbonatlösung gewaschen. Die durchsichtigen wäßrigen Flüssigkeiten wurden 5-mal mit je 10 $cm^3$ Äthylacetat gewaschen und ihr pH-Wert wurde auf 2 eingestellt. Es wurde zur Trockne eingedampft und der Rückstand wurde in Tetrahydrofuran aufgenommen, filtriert und zur Trockne eingedampft. Der ölige Rückstand wurde mit Diäthyläther zerrieben und unter Vakuum gesammelt. So wurde 0,2 g (R)-6β-[α-(2'-Formylhydrazino)-phenylacetamido]-penicillansäure mit einem Schmelzpunkt von 145,5 bis 147,8 °C (unter Zersetzung) und einem $[\alpha]_D$-Wert von + 117,6° (c = 1 in Tetrahydrofuran) erhalten.

## Beispiel 18

(R, S)-6β-[α-(1',2'-Diformylhydrazino)-thien-2"-ylacetamido]-penicillansäurepivaloyloxymethylester

Es wurde wie im Beispiel 12 beschrieben gearbeitet, jedoch als Ausgangsmaterial (R, S)-α-Hydrazinothien-2-ylacetylchloridhydrochlorid an Stelle des (R)-α-(1-Methylhydrazino)-phenylacetylchloridhydrochlorides verwendet. So wurde (R, S)-6β-[α-(1',2'-Diformylhydrazino)-thien-2"-ylacetamido]-penicillansäurepivaloyloxymethylester, welcher mit der nach dem Beispiel 6 erhaltenen Verbindung identisch war, erhalten.

Beispiel 19

(R, S)-6β-[α-(1′,2′-Diformylhydrazino)-phenylacetamido]-penicillansäurepivaloyloxymethylester

Es wurde wie im Beispiel 12 beschrieben gearbeitet, jedoch als Ausgangsmaterial (R)-α-Hydrazinophenylacetylchloridhydrochlorid an Stelle des (R)-α-(1-Methylhydrazino)-phenylacetylchloridhydrochlorides verwendet. So wurde (R)-6β-[α-(1′,2′-Diformylhydrazino)-phenylacetamido]-penicillansäurepivaloyloxymethylester, welcher mit der nach dem Beispiel 5 erhaltenen Verbindung identisch war, erhalten.

**Ansprüche** (für die Vertragsstaaten : BE, CH, DE, FR, GB, IT, LI, LU, NL, SE)

1. Formylderivate von Hydrazinomethylpenicillinen der allgemeinen Formel

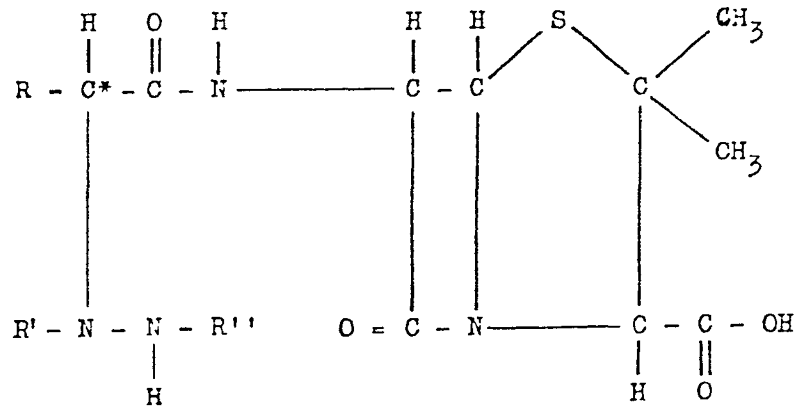

(I)

worin
R für eine Thienyl- oder Phenylgruppe steht,
R′ Wasserstoff, eine Formylgruppe oder eine Alkylgruppe mit 1 bis 3 Kohlenstoffatomen bedeutet,
R″ Wasserstoff oder eine Formylgruppe darstellt und
das Sternchen (*) ein Asymmetriezentrum des Moleküles darstellt,
mit der Maßgabe, daß mindestens eines der Symbole von R′ und R″ eine Formylgruppe darstellt,
in Form der getrennten Epimere oder von Mischungen derselben sowie ihre pharmazeutisch brauchbaren Salze und 1-(Äthoxycarbonyloxy)-äthyl- und Pivaloyloxymethylester.

2. Formylderivate von Hydrazinomethylpenicillinen nach Anspruch 1, dadurch gekennzeichnet, daß der Alkylrest, für den R′ stehen kann, ein solcher mit einem oder zwei, insbesondere einem Kohlenstoffatom(en) ist.

3. (R)-6β-[α-(2′-Formyl-1′-methylhydrazino)-phenylacetamido]-penicillansäure.

4. (R)-6β-[α-(1′,2′-Diformylhydrazino)-phenylacetamido]-penicillansäure.

5. (R)-6β-[α-(2′-Formylhydrazino)-phenylacetamido]-penicillansäure.

6. (R)-6β-[α-(2′-Formylhydrazino)-phenylacetamido]-penicillansäure-{1″-(äthoxycarbonyloxy)-äthyl}-ester.

7. Verfahren zur Herstellung der Verbindungen nach Anspruch 1 bis 6, dadurch gekennzeichnet, daß man in an sich bekannter Weise

a) Hydrazinomethylpenicilline der allgemeinen Formel

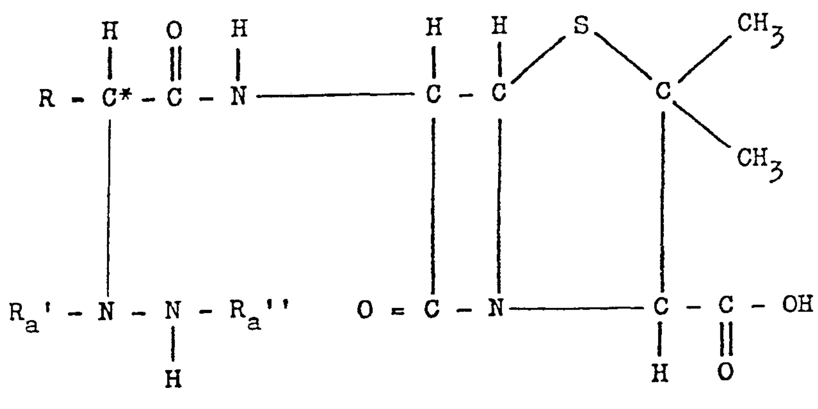

(II)

worin

R und das Sternchen (*) wie im Anspruch 1 festgelegt sind,

$R_a'$ für Wasserstoff, eine Alkylgruppe mit 1 bis 3 Kohlenstoffatomen oder eine übliche Schutzgruppe steht und

$R_a''$ Wasserstoff oder eine übliche Schutzgruppe darstellt,

wobei mindestens eines der Symbole von $R_a'$ und $R_a''$ keine Schutzgruppe bedeutet, in Form der getrennten Epimere oder von Mischungen derselben beziehungsweise Salze oder Ester derselben, gegebenenfalls nur mit üblicher Schutzgruppenfunktion der Estergruppe, unter wasserfreien Bedingungen mit dem gemischten Anhydrid von Essigsäure und Ameisensäure formyliert und die gegebenenfalls vorliegende(n) Schutzgruppe(n) entfernt oder

b) α-Hydrazinoessigsäurederivate der allgemeinen Formel

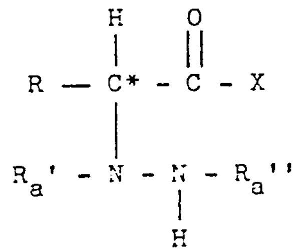

(III)

worin

R und das Sternchen (*) wie im Anspruch 1 festgelegt sind,

$R_a'$ für Wasserstoff, eine Alkylgruppe mit 1 bis 3 Kohlenstoffatomen oder eine übliche Schutzgruppe steht,

$R_a''$ Wasserstoff oder eine übliche Schutzgruppe bedeutet,

wobei mindestens eines der Symbole von $R_a'$ und $R_a''$ keine Schutzgruppe bedeutet, und

X eine Hydroxygruppe oder eine bei der anschließenden Kondensation oder vor dieser in sonstiger üblicher Weise abspaltbare übliche Schutzgruppe darstellt,

in Form der getrennten Epimere oder von Mischungen derselben, gegebenenfalls als Salz mit dem gemischten Anhydrid von Essigsäure und Ameisensäure unter wasserfreien Bedingungen formyliert und die so erhaltenen Formylderivate von α-Hydrazinoessigsäurederivaten der allgemeinen Formel

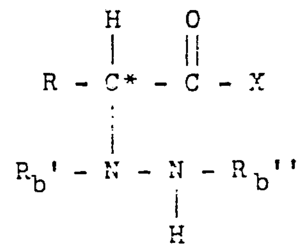

(IV)

worin

R und das Sternchen (*) wie im Anspruch 1 oder 2 festgelegt sind,

$R_b'$ für Wasserstoff, eine Formylgruppe, eine Alkylgruppe mit 1 bis 3 Kohlenstoffatomen oder eine übliche Schutzgruppe steht,

$R_b''$ Wasserstoff, eine Formylgruppe oder eine übliche Schutzgruppe bedeutet,

wobei mindestens eines der Symbole von $R_b'$ und $R_b''$ keine Schutzgruppe bedeutet und mindestens eines der Symbole von $R_b'$ und $R_b''$ eine Formylgruppe darstellt, und

X wie vorstehend festgelegt ist,

gegebenenfalls nach ihrer Überführung in Salze, mit 6-Aminopenicillansäure als solcher oder als Salz oder in Form von Estern, gegebenenfalls nur mit üblicher Schutzgruppenfunktion der Estergruppe, kondensiert sowie die gegebenenfalls vorliegende(n) Schutzgruppe(n) vor und/oder nach dieser Umsetzung entfernt oder

c) α-Hydrazinoessigsäurederivate der allgemeinen Formel

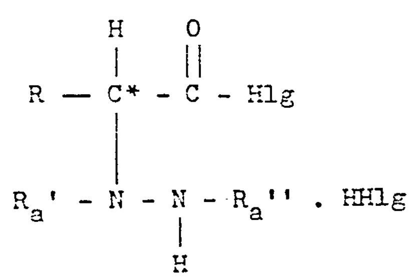

(V)

worin

R und das Sternchen (*)' wie im Anspruch 1 festgelegt sind,

$R_a'$ für Wasserstoff, eine Alkylgruppe mit 1 bis 3 Kohlenstoffatomen oder eine übliche Schutzgruppe steht,

$R_a''$ Wasserstoff oder eine übliche Schutzgruppe bedeutet,

wobei mindestens eines der Symbole von $R_a'$ und $R_a''$ keine Schutzgruppe bedeutet, und

Hlg für Chlor oder Brom steht,

in Form der getrennten Epimere oder von Mischungen derselben, mit 6-Aminopenicillansäure als solcher oder als Salz oder in Form von Estern, gegebenenfalls nur mit üblicher Schutzgruppenfunktion der Estergruppe, kondensiert und die so erhaltenen Hydrazinomethylpenicilline der allgemeinen Formel

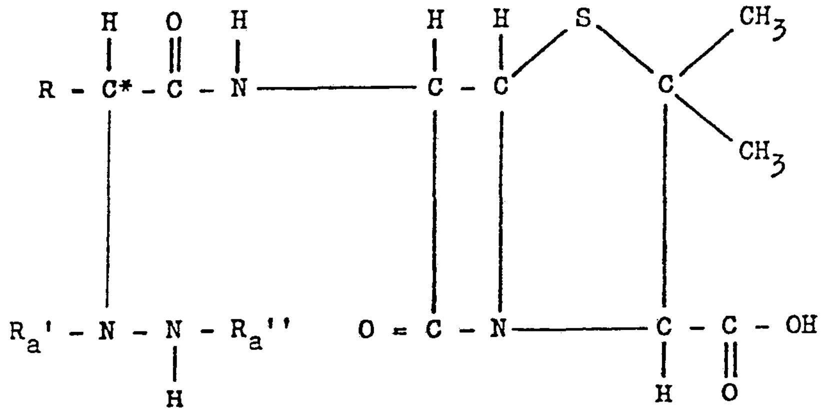

(II)

worin R, das Sternchen (*), $R_a'$ und $R_a''$ wie vorstehend festgelegt sind, mit dem gemischten Anhydrid von Essigsäure und Ameisensäure unter wasserfreien Bedingungen formyliert sowie die gegebenenfalls vorliegende(n) Schutzgruppe(n) entfernt,

worauf man in an sich bekannter Weise gegebenenfalls die so erhaltenen Formylderivate von Hydrazinomethylpenicillinen der allgemeinen Formel I mit Saüren in pharmazeutisch brauchbare Säureadditionssalze überführt oder verestert beziehungsweise gegebenenfalls die so erhaltenen Säureadditionssalze der Formylderivate von Hydrazinomethylpenicillinen der allgemeinen Formel I in die freien Formylderivate von Hydrazinomethylpenicillinen der allgemeinen Formel I oder in andere Salze überführt beziehungsweise gegebenenfalls die so erhaltenen Ester der Formylderivate von Hydrazinomethylpenicillinen der allgemeinen Formel I in die freien Formylderivate von Hydrazinomethylpenicillinen der allgemeinen Formel I überführt und/oder gegebenenfalls die so erhaltenen Mischungen der Epimere der Formylderivate von Hydrazinomethylpenicillinen der allgemeinen Formel I in die Epimere spaltet beziehungsweise die so erhaltenen Epimere der Formylderivate von Hydrazinomethylpenicillinen der allgemeinen Formel I racemisiert.

8. Arzneimittel, gekennzeichnet durch einen Gehalt an einer oder mehreren Verbindungen nach Anspruch 1 bis 6 als Wirkstoff(en), gegebenenfalls zusammen mit einem oder mehreren üblichen pharmazeutischen Konfektionierungsmitteln.

**Ansprüche** (für den Vertragsstaat AT)

1. Verfahren zur Herstellung von Formylderivaten von Hydrazinomethylpenicillinen der allgemeinen Formel

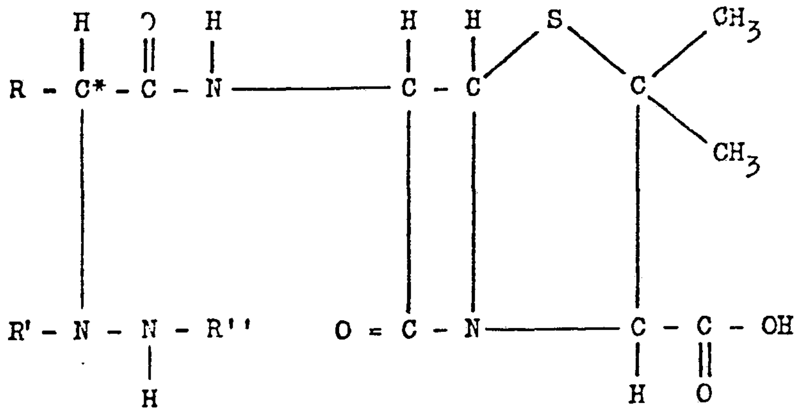

(I)

worin

R für eine Thienyl- oder Phenylgruppe steht,

R' Wasserstoff, eine Formylgruppe oder eine Alkylgruppe mit 1 bis 3 Kohlenstoffatomen bedeutet,

R" Wasserstoff oder eine Formylgruppe darstellt und

das Sternchen (*) ein Asymmetriezentrum des Moleküles darstellt,

mit der Maßgabe, daß mindestens eines der Symbole von R' und R" eine Formylgruppe darstellt, in Form der getrennten Epimere oder von Mischungen derselben sowie ihren pharmazeutisch brauchbaren Salzen und 1-(Äthoxycarbonyloxy)-äthyl- und Pivaloyloxymethylestern, dadurch gekennzeichnet, daß man in an sich bekannter Weise

a) Hydrazinomethylpenicilline der allgemeinen Formel

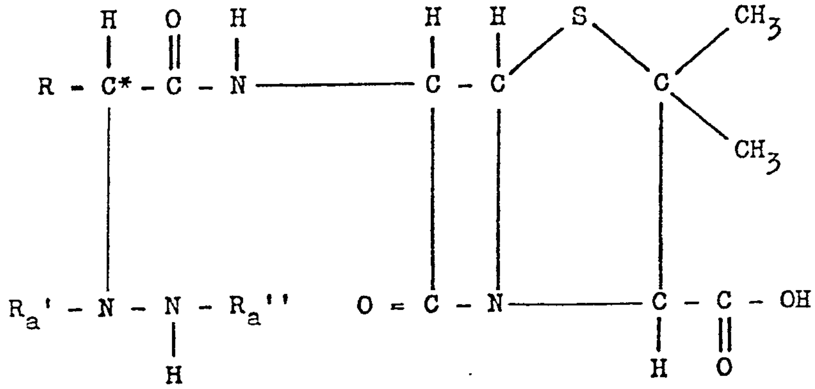

(II)

worin

R und das Sternchen (*) wie oben festgelegt sind,

$R_a'$ für Wasserstoff, eine Alkylgruppe mit 1 bis 3 Kohlenstoffatomen oder eine übliche Schutzgruppe steht und

$R_a''$ Wasserstoff oder eine übliche Schutzgruppe darstellt,

wobei mindestens eines der Symbole von $R_a'$ und $R_a''$ keine Schutzgruppe bedeutet, in Form der getrennten Epimere oder von Mischungen derselben beziehungsweise Salze oder Ester derselben, gegebenenfalls nur mit üblicher Schutzgruppenfunktion der Estergruppe, unter wasserfreien Bedingungen mit dem gemischten Anhydrid von Essigsäure und Ameisensäure formyliert und die gegebenenfalls vorliegende(n) Schutzgruppe(n) entfernt oder

b) α-Hydrazinoessigsäurederivate der allgemeinen Formel

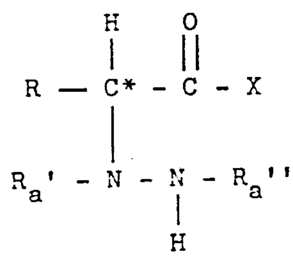

(III)

worin

R und das Sternchen (*) wie oben festgelegt sind,

$R_a'$ für Wasserstoff, eine Alkylgruppe mit 1 bis 3 Kohlenstoffatomen oder eine übliche Schutzgruppe steht,

$R_a''$ Wasserstoff oder eine übliche Schutzgruppe bedeutet, wobei mindestens eines der Symbole von $R_a'$ und $R_a''$ keine Schutzgruppe bedeutet, und

X eine Hydroxygruppe oder eine bei der anschließenden Kondensation oder vor dieser in sonstiger üblicher Weise abspaltbare übliche Schutzgruppe darstellt,

in Form der getrennten Epimere oder von Mischungen derselben, gegebenenfalls als Salz, mit dem gemischten Anhydrid von Essigsäure und Ameisensäure unter wasserfreien Bedingungen formyliert und die so erhaltenen Formylderivate von α-Hydrazinoessigsäurederivaten der allgemeinen Formel

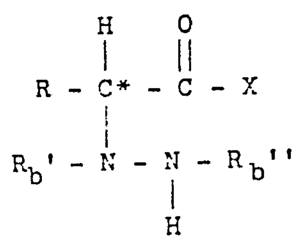

(IV)

worin

R und das Sternchen (*) wie oben festgelegt sind,

$R_b'$ für Wasserstoff, eine Formylgruppe, eine Alkylgruppe mit 1 bis 3 Kohlenstoffatomen oder eine übliche Schutzgruppe steht,

$R_b''$ Wasserstoff, eine Formylgruppe oder eine übliche Schutzgruppe bedeutet, wobei mindestens eines der Symbole von $R_b'$ und $R_b''$ keine Schutzgruppe bedeutet und mindestens eines der Symbole von $R_b'$ und Rb" eine Formylgruppe darstellt und

X wie vorstehend festgelegt ist,

gegebenenfalls nach ihrer Überführung in Salze, mit 6-Aminopenicillansäure als solcher oder als Salz oder in Form von Estern, gegebenenfalls nur mit üblicher Schutzgruppenfunktion der Estergruppen kondensiert sowie die gegebenenfalls vorliegende(n) Schutzgruppe(n) vor und/oder nach dieser Umsetzung entfernt oder

c) α-Hydrazinoessigsäurederivate der allgemeinen Formel

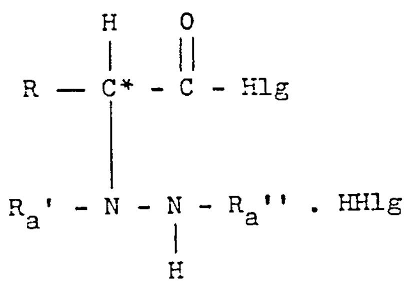

(V)

worin

R und das Sternchen (*) wie oben festgelegt sind,

$R_a'$ für Wasserstoff, eine Alkylgruppe mit 1 bis 3 Kohlenstoffatomen oder eine übliche Schutzgruppe steht,

$R_a''$ Wasserstoff oder eine übliche Schutzgruppe bedeutet, wobei mindestens eines der Symbole von $R_a'$ und $R_a''$ keine Schutzgruppe bedeutet, und

Hlg für Chlor oder Brom steht,

in Form der getrennten Epimere oder von Mischungen derselben, mit 6-Aminopenicillansäure als solcher oder als Salz oder in Form von Estern, gegebenenfalls nur mit üblicher Schutzgruppenfunktion de Estergruppe, kondensiert und die so erhaltenen Hydrazinomethylpenicilline der allgemeinen Formel

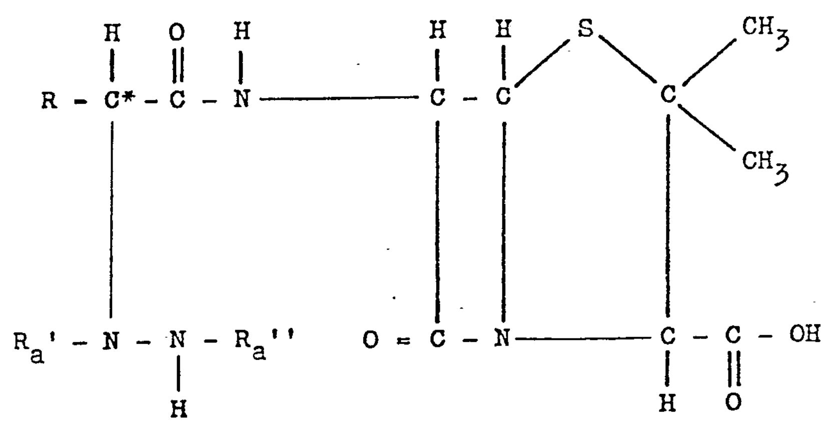

(II)

worin R, das Sternchen (*), $R_a'$ und $R_a''$ wie vorstehend festgelegt sind, mit dem gemischten Anhydrid von Essigsäure und Ameisensäure unter wasserfreien Bedingungen formyliert sowie die gegebenenfalls vorliegende(n) Schutzgruppe(n) entfernt,

worauf man in an sich bekannter Weise gegebenenfalls die so erhaltenen Formylderivate von Hydrazinomethylpenicillinen der allgemeinen Formel I mit Säuren in pharmazeutisch brauchbare Säureadditionssalze überführt oder verestert beziehungsweise gegebenenfalls die so erhaltenen Säureadditionssalze der Formylderivate von Hydrazinomethylpenicillinen der allgemeinen Formel I in die freien Formylderivate von Hydrazinomethylpenicillinen der allgemeinen Formel I oder in andere Salze überführt beziehungsweise gegebenenfalls die so erhaltenen Ester der Formylderivate von Hydrazinomethylpenicillinen der allgemeinen Formel I in die freien Formylderivate von Hydrazinomethylpenicillinen der allgemeinen Formel I überführt und/oder gegebenenfalls die so erhaltenen Mischungen der Epimere der Formylderivate von Hydrazinomethylpenicillinen der allgemeinen Formel I in die Epimere spaltet beziehungsweise die so erhaltenen Epimere der Formylderivate von Hydrazinomethylpenicillinen der allgemeinen Formel I racemisiert.

2. Verfahren nach Anspruch 1, dadurch gekennzeichnet, daß man als Alkylrest, für den R′ stehen kann, einen solchen mit einem oder zwei insbesondere einem Kohlenstoffatom(en) verwendet.

3. Verfahren nach Anspruch 1 oder 2, dadurch gekennzeichnet, daß man (R)-6β-[α-(2′-Formyl-1′-methylhydrazino)-phenylacetamido]-penicillansäure herstellt.

4. Verfahren nach Anspruch 1 oder 2, dadurch gekennzeichnet, daß man (R)-6β-[α-(1′,2′-Diformylhydrazino)-phenylacetamido]-penicillansäure herstellt.

5. Verfahren nach Anspruch 1 oder 2, dadurch gekennzeichnet, daß man (R)-6β-[α-2′-Formylhydrazino)-phenylacetamido]-penicillansäure herstellt.

6. Verfahren nach Anspruch 1 oder 2, dadurch gekennzeichnet, daß man (R)-6β-[α-(2′-Formylhydrazino)-phenylacetamido]-penicillansäure-{1″-(äthoxy-carbonyloxy)-äthyl}-ester herstellt.

**Claims** (for the Contracting States : BE, CH, DE, FR, GB, IT, LI, LU, NL, SE)

1. Formyl derivatives of hydrazinomethylpenicillins having the general formula

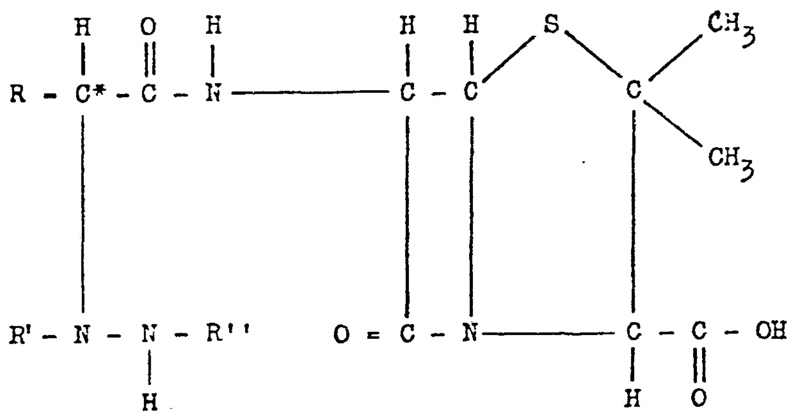

(I)

wherein

R represents a thienyl or phenyl group,

R′ means hydrogen, a formyl group or an alkyl group having from 1 to 3 carbon atoms,

R″ represents hydrogen or a formyl group and

the asterisk (*) represents an asymmetry centre of the molecule

with the definition that at least one of the symbols R′ and R″ represents a formyl group, in the form of the separated epimers or of mixtures of them as well as their pharmaceutically usable salts and 1-(ethoxycarbonyloxy)-ethyl and pivaloyloxymethyl esters.

2. Formyl derivatives of hydrazinomethylpenicillins according to claim 1, characterized in that the alkyl radical which can be represented by R′ is such one having one or two, particularly one carbon atom(s).

3. (R)-6β-[α-(2′-Formyl-1′-methylhydrazino)-phenylacetamido]-penicillanic acid.

4. (R)-6β-[α-(1′,2′-Diformylhydrazino)-phenylacetamido]-penicillanic acid.

5. (R)-6β-[α-(2′-Formylhydrazino)-phenylacetamido]-penicillanic acid.

6. 1″-(Ethoxycarbonyloxy)-ethyl ester of (R)-6β-[α-(2′-formylhydrazino)-phenylacetamido]-penicillanic acid.

7. A process for preparing the compounds according to claims 1 to 6, characterized in that in a manner known per se

a) one formylates hydrazinomethylpenicillins having the general formula

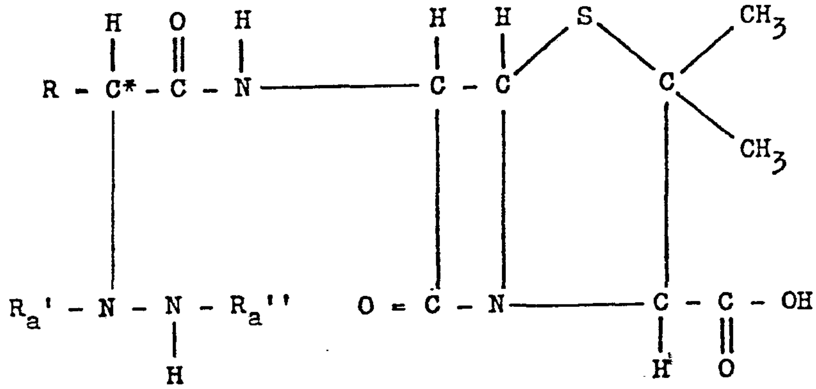

(II)

wherein
R and the asterisk (*) are as defined in claim 1,
$R_a'$ represents hydrogen, an alkyl group having from 1 to 3 carbon atoms or an usual protective group and
$R_a''$ represents hydrogen of an usual protective group
at least one of the symbols $R_a'$ and $R_a''$ meaning no protective group in the form of the separated epimers or of mixtures of them of salts or esters of them, respectively, optionally only with an usual protective group function of the ester group, under anhydrous conditions with the mixed anhydride of acetic acid and formic acid and one eliminates the protective group(s) optionally present or
b) one formylates derivatives of α-hydrazinoacetic acid having the general formula

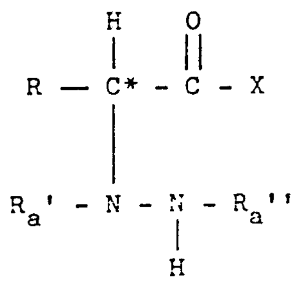

(III)

wherein
R and the asterisk (*) are as defined in claim 1,
$R_a'$ represents hydrogen, an alkyl group having from 1 to 3 carbon atoms or an usual protective group,
$R_a''$ means hydrogen or an usual protective group
at least one of the symbols $R_a'$ and $R_a''$ meaning no protective group and
X represents a hydroxy group or an usual protective group splittable off in the subsequent condensation or before it in another usual way in the form of the separated epimers or of mixtures of them, optionally as a salt, with the mixed anhydride of acetic acid and formic acid unter anhydrous conditions and one condenses the thus obtained formyl derivatives of derivatives of α-hydrazinoacetic acid having the general formula

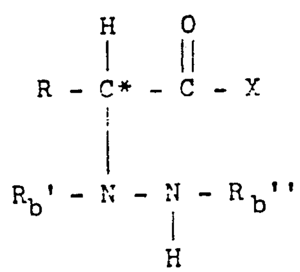

(IV)

wherein
R and the asterisk (*) are as defined in claim 1 or 2,
$R_b'$ represents hydrogen, a formyl group, an alkyl group having from 1 to 3 carbon atoms or an usual protective group,
$R_b''$ means hydrogen, a formyl group or an usual protective group
at least one of the symbols $R_b'$ and $R_b''$ meaning no protective group and at least one of the symbols $R_b'$ and $R_b''$ represents a formyl group and
X is as defined in the foregoing,
optionally after their conversion into salts, with 6-aminopenicillanic acid as such or as salt of in the form of esters, optionally only with usual protective group function of the ester group, as well as one eliminates the protective group(s) optionally present before and/or after this reaction or
c) one condenses derivatives of α-hydrazinoacetic acid having the general formula

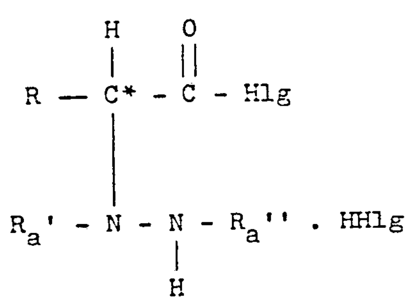

(V)

wherein

R and the asterisk (*) are as defined in claim 1,

$R_a'$ represents hydrogen, an alkyl group having from 1 to 3 carbon atoms of an usual protective group,

$R_a''$ means hydrogen or an usual protective group

at least one of the symbols $R_a'$ and $R_a''$ meaning no protective group and

Hlg represents chlorine of bromine,

in the form of the separated epimers or of mixtures of them, with 6-aminopenicillanic acid as such or as salt of in the form of esters, optionally with usual protective group function of the ester group, and one formylates the thus obtained hydrazinomethylpenicillins having the general formula

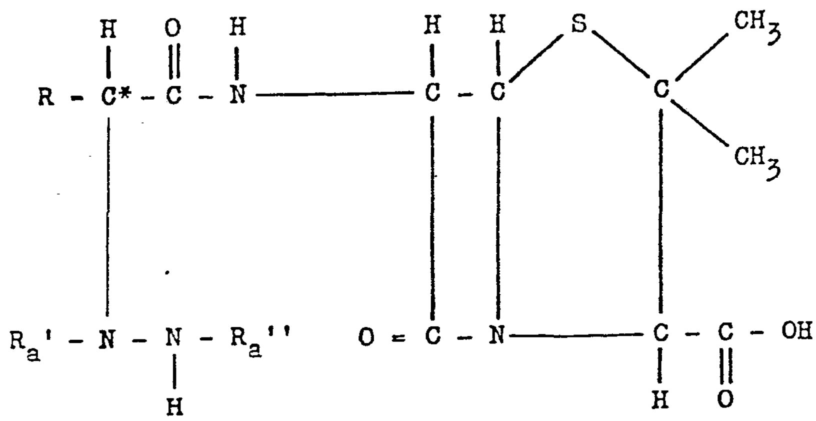

(II)

wherein R, the asterisk (*), $R_a'$ and $R_a''$ are defined as in the foregoing with the mixed anhydride of acetic acid and formic acid under anhydrous conditions as well as one eliminates the protective group(s) optionally present,

whereupon in a manner known per se optionally one converts the thus obtained formyl derivatives of hydrazinomethylpenicillins having the general formula I with acids into pharmaceutically usable acid addition salts or esterifies them of optionally one converts the thus obtained acid addition salts of the formyl derivatives of hydrazinomethylpenicillins having the general formula I into the free formyl derivatives of hydrazinomethylpenicillins having the general formula I or into other salts, respectively, or optionally one converts the thus obtained esters of the formyl derivatives of hydrazinomethylpenicillins having the general formula I into the free formyl derivatives of hydrazinomethylpenicillins having the general formula I, respectively, and/or optionally one separates the thus obtained mixtures of the epimers of the formyl derivatives of hydrazinomethylpenicillins having the general formula I into the epimers or one racemises the thus obtained epimers of the formyl derivatives of hydrazinomethylpenicillins having the general formula I, respectively.

8. Medicaments, characterized by a content of one or several compounds according to claims 1 to 6 as [an] active principle(s), optionally together with one or several usual pharmaceutical confectioning agents.

**Claims** (for the Contracting State AT)

1. A process for preparing formyl derivatives of hydrazinomethylpenicillins having the general formula

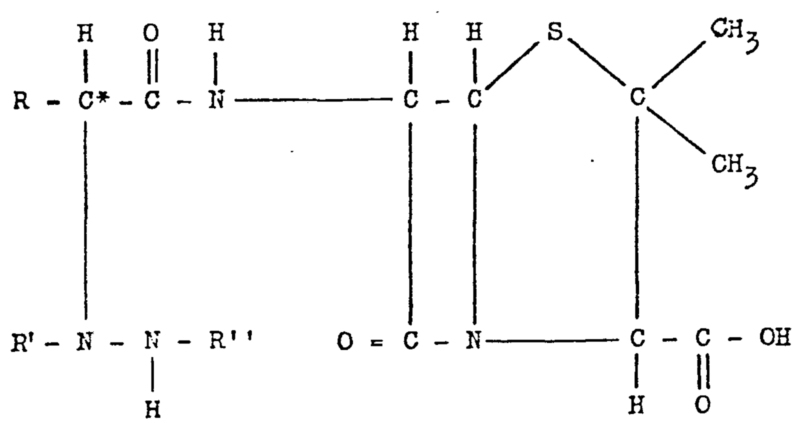

(I)

wherein

R represents a thienyl or phenyl group,

R′ means hydrogen, a formyl group or an alkyl group having from 1 to 3 carbon atoms,

R″ represents hydrogen or a formyl group and

the asterisk (*) represents an asymmetry centre of the molecule with the definition that at least one of the symbols R′ and R″ represents a formyl group,

in the form of the separated epimers or of mixtures of them as well as their pharmaceutically usable salts and 1-(ethoxycarbonyloxy)-ethyl and pivaloyloxymethyl esters, characterized in that in a manner known per se

a) one formylates hydrazinomethylpenicillins having the general formula

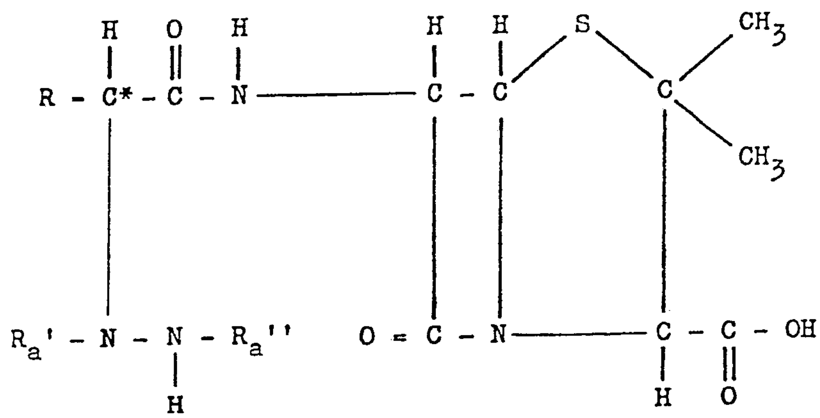

(II)

wherein

R and the asterisk (*) are as above defined,

$R_a'$ represents hydrogen, an alkyl group having from 1 to 3 carbon atoms or an usual protective group and

$R_a''$ represents hydrogen or an usual protective group

at least one of the symbols $R_a'$ and $R_a''$ meaning no protective group

in the form of the separated epimers or of mixtures of them or salt or esters of them, respectively, optionally only with an usual protective group function of the ester group, under anhydrous conditions with the mixed anhydride of acetic acid and formic acid and one eliminates the protective group(s) optionally present or

b) one formylates derivatives of α-hydrazinoacetic acid having the general formula

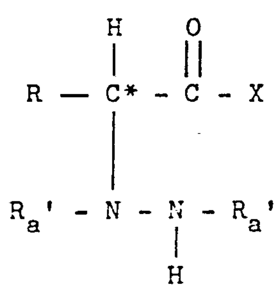

(III)

wherein

R and the asterisk (*) are as above defined,

$R_a'$ represents hydrogen, an alkyl group having from 1 to 3 carbon atoms or an usual protective group,

$R_a''$ means hydrogen or an usual protective group

at least one of the symbols $R_a'$ and $R_a''$ meaning no protective group and

X represents a hydroxy group or an usual protective group splittable off in the subsequent condensation or before it in another usual way

in the form to the separated epimers or of mixtures of them, optionally as a salt, with the mixed anhydride of acetic acid and formic acid under anhydrous conditions and one condenses the thus obtained formyl derivatives of derivatives of α-hydrazinoacetic acid having the general formula

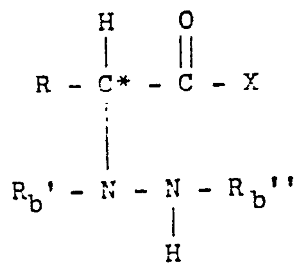

(IV)

wherein

R and the asterisk (*) are as above defined,

$R_b'$ represents hydrogen, a formyl group, an alkyl group having from 1 to 3 carbon atoms or an usual protective group,

$R_b''$ means hydrogen, a formyl group or an usual protective group at least one on the symbols $R_b'$ and $R_b''$ meaning no protective group and at least one of the symbols $R_b'$ and $R_b''$ represents a formyl group and

X is as defined in the foregoing,

optionally after their conversion into salts, with 6-aminopenicillanic acid as such or as salt or in the form of esters, optionally only with usual protective group function of the ester group, as well as one eliminates the protective group(s) optionally present before and/or after this reaction or

c) one condenses derivatives of α-hydrazinoacetic acid having the general formula

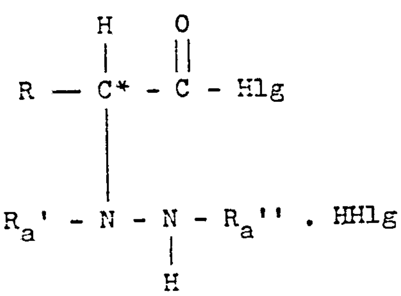

(V)

wherein

R and the asterisk (*) are as above defined,

$R_a'$ represents hydrogen, an alkyl group having from 1 to 3 carbon atoms or an usual protective group,

$R_a''$ means hydrogen or an usual protective group

at least one of the symbols $R_a'$ and $R_a''$ meaning no protective group and

Hlg represents chlorine or bromine,

in the form of the separated epimers or of mixtures of them, with 6-aminopenicillanic acid as such or as salt or in the form of esters, optionally only with usual protective group function of the ester group, and one formylates the thus obtained hydrazinomethylpenicillins having the general formula

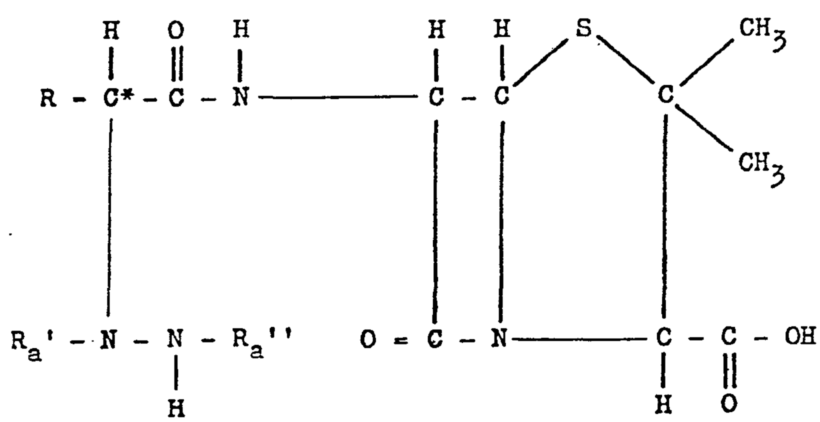

(II)

wherein R, the asterisk (*), $R_a'$ and $R_a''$ are defined as in the foregoing with the mixed anhydride of acetic acid and formic acid under anhydrous conditions as well as one eliminates the protective group(s) optionally present,

whereupon in a manner known per se optionally one converts the thus obtained formyl derivatives of hydrazinomethylpenicillins having the general formula I with acids into pharmaceutically usable acid addition salts or esterifies them or optionally one converts the thus obtained acid addition salts of the formyl derivatives of hydrazinomethyl-penicillins having the general formula I into the free formyl derivatives of hydrazinomethylpenicillins having the general formula I or into other salts, respectively, or optionally one converts the thus obtained esters of the formyl derivatives of hydrazinomethylpenicillins having the general formula I into the free formyl derivatives of hydrazinomethylpenicillins having the general formula I, respectively, and/or optionally one separates the thus obtained mixtures of the epimers of the formyl derivatives of hydrazinomethylpenicillins having the general formula I into the epimers or one racemises the thus obtained epimers of the formyl derivatives of hydrazinomethylpenicillins having the general formula I, respectively.

2. A process according to claim 1, characterized in that one uses as an alkyl radical which can be represented by R′ such one having one or two, particularly one carbon atom(s).

3. A process according to claim 1 or 2, characterized in that one prepares (R)-6β-[α-(2′-formyl-1′-methylhydrazino)-phenylacetamido]-penicillanic acid.

4. A process according to claim 1 or 2, characterized in taht one prepares (R)-6β-[α-(1′,2′-diformylhydrazino)-phenylacetamido]-penicillanic acid.

5. A process according to claim 1 or 2, characterized in that one prepares (R)-6β-[α-(2′-formylhydrazino)-phenylacetamido]-penicillanic acid.

6. A process according to claim 1 or 2, characterized in that one prepares 1″-ethoxycarbonyloxy)-ethyl ester of (R)-6β-[α-(2′-formylhydrazino)-phenylacetamido]-penicillanic acid.

**Revendications** (pour les Etats Contractants : BE, CH, DE, FR, GB, LT, LI, LU, NL, SE)

1. Dérivés formylés d'hydrazinométhylpénicillines de formule générale

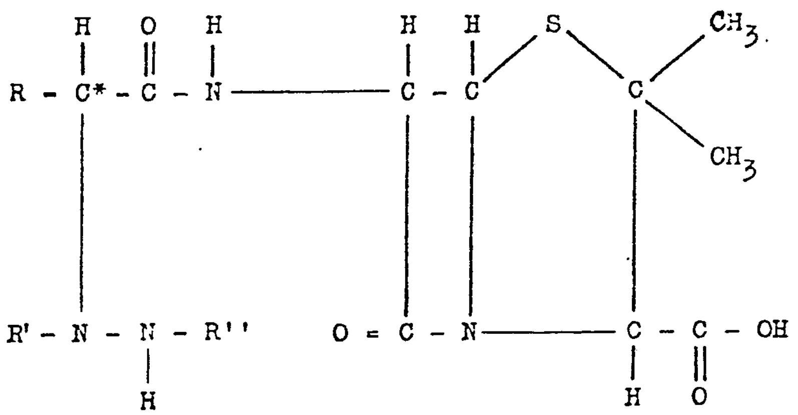

(I)

dans laquelle

R représente un groupe thiényle ou phényle,

R′ représente l'hydrogène, un groupe formyle ou un groupe alkyle de 1 à 3 atomes de carbone,

R″ représente l'hydrogène ou un groupe formyle et l'astérisque (*) représente un centre d'asymétrie de la molécule,

sous réserve qu'au moins un des symboles R′ et R″ représente un groupe formyle, sous la forme des épimères séparés ou de mélanges de ceux-ci ainsi que leurs sels et esters de 1-(éthoxycarbonyloxy)-éthyle et de pivaloyloxy-méthyle pharmaceutiquement utilisables.

2. Dérivés formylés d'hydrazinométhylpénicillines selon la revendication 1, caractérisés par le fait que le radical alkyle que peut représenter R′ est un radical contenant un ou deux atomes de carbone, en particulier un seul atome de carbone.

3. Acide (R)-6β-[α-(2′-formyl-1′-méthylhydrazino)-phénylacétamido]-pénicillanique.

4. Acide (R)-6β-[α-(1′,2′-diformylhydrazino)-phénylacétamido]-pénicillanique.

5. Acide (R)-6β-[α-(2′-formylhydrazino)-phénylacétamido]-pénicillanique.

6. (R)-6β-[α-(2′-formylhydrazino)-phénylacétamido]-pénicillanate de 1″-(éthoxycarbonyloxy)-éthyle.

7. Procédé de préparation des composés selon les revendications 1 à 6, caractérisé par le fait que, de manière en elle-même connue :

a) on formyle, en milieu anhydre, avec l'anhydride mixte de l'acide acétique et de l'acide formique, des hydrazinométhylpénicillines de formule générale

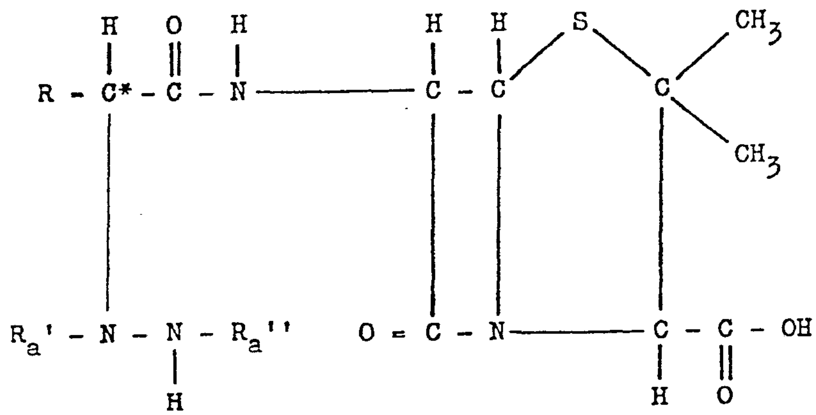

(II)

dans laquelle

R et l'astérisque (*) sont tels que définis à la revendication 1,

$R_a'$ représente l'hydrogène, un groupe alkyle de 1 à 3 atomes de carbone ou un groupe protecteur usuel et

$R_a''$ représente l'hydrogène ou un groupe protecteur usuel, au moins un des symboles $R_a'$ et $R_a''$ ne représentant pas un groupe protecteur,

sous la forme des épimères séparés ou de mélanges de ceux-ci ou de sels ou esters de celle-ci, le groupe ester n'ayant éventuellement qu'une fonction usuelle de groupe protecteur, et on élimine le ou les groupe(s) protecteur(s) éventuellement présent(s), ou

b) on formyle, en milieu anhydre, avec l'anhydride mixte de l'acide acétique et de l'acide formique, des dérivés d'acide α-hydrazinoacétique de la formule générale

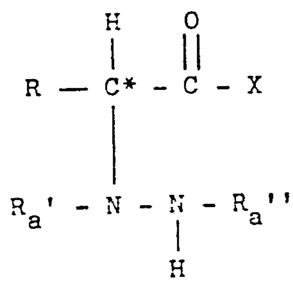

(III)

dans laquelle

R et l'astérique (*) sont tels que définis à la revendication 1,

$R_a'$ représente l'hydrogène, un groupe alkyle de 1 à 3 atomes de carbone ou un groupe protecteur usuel,

$R_a''$ représente l'hydrogène ou un groupe protecteur usuel, au moins un des symboles $R_a'$ et $R_a''$ ne représentant pas un groupe protecteur et

X représente un groupe hydroxyle ou un groupe protecteur usuel pouvant être être éliminé lors de la condensation qui suit ou avant celle-ci d'une manière usuelle,

sous la forme des épimères séparés ou de mélanges de ceux-ci, éventuellement sous forme de sel, et on condense les dérivés formylés de dérivés d'acide α-hydrazinoacétique, ainsi obtenus, de formule générale

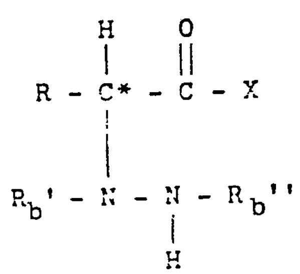

(IV)

dans laquelle

R et l'astérisque (*) sont tels que définis à l'une des revendications 1 et 2,

$R_b'$ représente l'hydrogène, un groupe formyle, un groupe alkyle de 1 à 3 atomes de carbone ou un groupe protecteur usuel,

$R_b''$ représente l'hydrogène, un groupe formyle ou un groupe protecteur usuel, au moins un des symboles $R_b'$ et $R_b''$ représentant un groupe formyle, et

X étant tel que défini plus haut,

éventuellement après leur conversion en sels, avec l'acide 6-aminopénicillanique, tel quel ou sous forme de sel ou sous forme d'esters, le groupe ester n'ayant éventuellement qu'une fonction usuelle de groupe protecteur, et on élimine, avant et/ou après cette réaction, le ou les groupe(s) protecteur(s) éventuellement présent(s), ou

c) on condense des dérivés d'acide α-hydrazinoacétique de formule générale

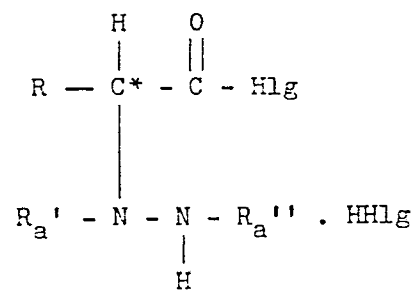

(V)

dans laquelle

R et l'astérisque (*) sont tels que définis à la revendication 1,

$R_a'$ représente l'hydrogène, un groupe alkyle de 1 à 3 atomes de carbone ou un groupe protecteur usuel,

$R_a''$ représente l'hydrogène ou un groupe protecteur usuel, au moins un des symboles $R_a'$ et $R_a''$ ne représentant pas un groupe protecteur, et

Hlg représente le chlore ou le brome,

sous la forme des épimères séparés ou de mélanges de ceux-ci, avec l'acide 6-aminopénicillanique, tel quel ou sous forme de sel ou sous forme d'esters, le groupe ester n'ayant éventuellement qu'une fonction usuelle de groupe protecteur, et on formyle, en milieu anhydre, avec l'anhydride mixte de l'acide acétique et de l'acide formique, les hydrazinométhylpénicillines ainsi obtenues, de formule générale

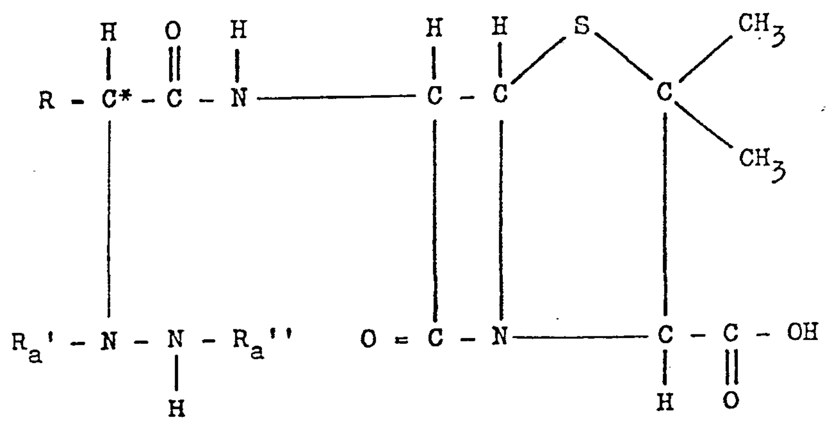

(II)

dans laquelle R, l'astérisque (*), $R_a'$ et $R_a''$ sont tels que définis plus haut, et on élimine le ou les groupe(s) protecteur(s) éventuellement présent(s),

après quoi, de manière en elle-même connue, on convertit éventuellement les dérivés formylés d'hydrazinométhylpénycillines ainsi obtenus, de la formule générale I, en sels d'addition d'acide pharmaceutiquement utilisables, au moyen d'acides, ou on les estérifie, ou éventuellement, on convertit les sels d'addition d'acide des dérivés formylés d'hydrazinométhylpénicillines de la formule générale I, ainsi obtenus, en dérivés formylés libres d'hydrazinométhylpénicillines de la formule générale I ou en d'autres sels, ou éventuellement on convertit les esters de dérivés formylés d'hydrazinométhylpénicillines de la formule générale I, ainsi obtenus, en dérivés formylés libres d'hydrazinométhylpénicillines de la formule générale I et/ou éventuellement, on dédouble en épimères les mélanges d'épimères de dérivés formylés d'hydrozinométhylpénicillines de la formule générale I, ainsi obtenus, ou on racémise les épimères de dérivés formylés dhydrazinométhylpénicillines de la formule générale I, ainsi obtenus.

8. Médicaments caractérisés par une teneur en un ou plusieurs composés selon les revendications 1 à 6 comme substance actives, éventuellement en même temps qu'un ou plusieurs agents de conditionnement pharmaceutiques usuels

**Revendications** (pour l'Etat contractant AT)

1. Procédé de préparation des dérivés formylés d'hydrazinométhylphénicillines de formule générale

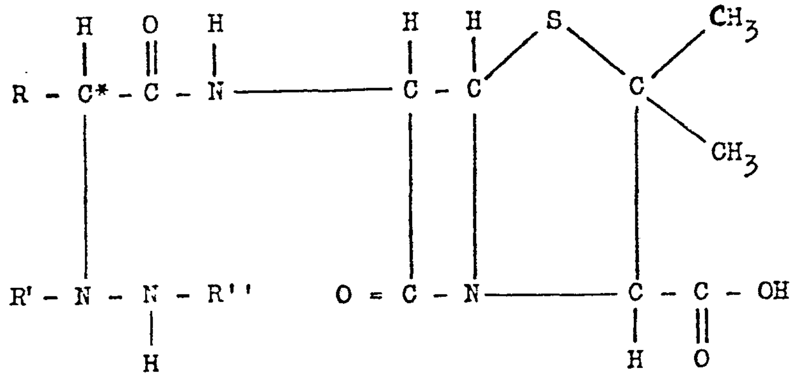

(I)

dans laquelle
R représente un groupe thiényle ou phényle,
R' représente l'hydrogène, un groupe formyle ou un groupe alkyle de 1 à 3 atomes de carbone,
R" représente l'hydrogène ou un groupe formyle et l'astérisque (*) représente un centre d'asymétrie de la molécule,
sous réserve qu'au moins un des symboles R' et R" représente un groupe formyle,
sous la forme des épimères séparés ou de mélanges de ceux-ci ainsi que leurs sels et esters de 1-(éthoxycarbonyloxyl)-éthyle et de pivaloyloxy-méthyle pharmaceutiquement utilisables, caractérisé par le fait que, de manière en elle-même connue :

a) on formyle, en milieu anhydre avec l'anhydride mixte de l'acide acétique et de l'acide formique, des hydrazinométhylpénicillines de formule générale

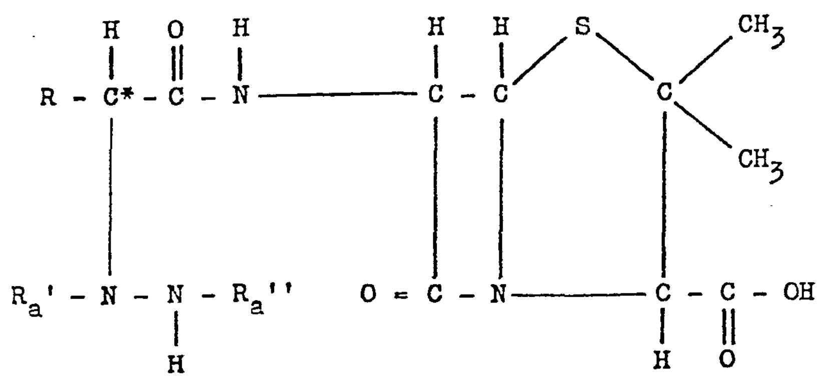

(II)

dans laquelle
R et l'astérisque (*) sont tels que définis plus haut,
$R_a'$ représente l'hydrogène, un groupe alkyle de 1 à 3 atomes de carbone ou un groupe protecteur usuel et
$R_a''$ représente l'hydrogène ou un groupe protecteur usuel, au moins un des symboles $R_a'$ et $R_a''$ ne représentant pas un groupe protecteur,
sous la forme des épimères séparés ou de mélanges de ceux-ci ou de sels ou esters de celle-ci, le groupe ester n'ayant éventuellement qu'une fonction usuelle de groupe protecteur, et on élimine le ou les groupe(s) protecteur(s) éventuellement présent(s), ou

b) on formyle, en milieu anhydre, avec l'anhydride mixte de l'acide acétique et de l'acide formique, des dérivés d'acide α-hydrazinoacétique de la formule générale

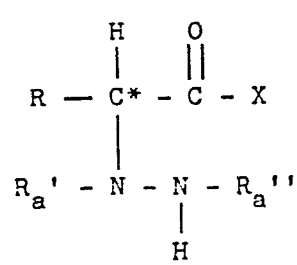

(III)

dans laquelle

R et l'astérisque (*) sont tels que définis plus haut,

$R_a'$ représente l'hydrogène, un groupe alkyle de 1 à 3 atomes de carbone ou un groupe protecteur usuel,

$R_a''$ représente l'hydrogène ou un groupe protecteur usuel, au moins un des symboles $R_a'$ et $R_a''$ ne représentant pas un groupe protecteur et

X représente un groupe hydroxyle ou un groupe protecteur usuel pouvant être éliminé lors de la condensation qui suit ou avant celle-ci d'une manière usuelle,

sous la forme des épimères séparés ou de mélanges de ceux-ci, éventuellement sous forme de sel, et on condense les dérivés formylés de dérivés d'acide α-hydrazinoacétique, ainsi obtenus, de formule générale

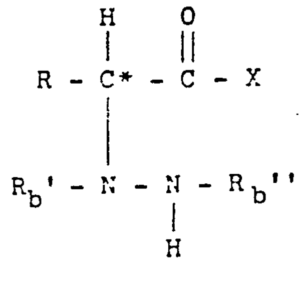

, (IV)

dans laquelle

R et l'astérisque (*) sont tels que définis plus haut,

$R_b'$ représente l'hydrogène, un groupe formyle, un groupe alkyle de 1 à 3 atomes de carbone ou un groupe protecteur usuel,

$R_b''$ représente l'hydrogène, un groupe formyle ou un groupe protecteur usuel,

au moins un des symboles $R_b'$ et $R_b''$ ne représentant pas un groupe protecteur et au moins un des symboles $R_b'$ et $R_b''$ représentant un groupe formyle, et

X étant tel que défini plus haut,

éventuellement après leur conversion en sels, avec l'acide 6-aminopénicillanique, tel quel ou sous forme de sel ou sous forme d'esters, le groupe ester n'ayant éventuellement qu'une fonction usuelle de groupe protecteur, et on élimine, avant et/ou après cette réaction, le ou les groupe(s) protecteur(s) éventuellement présent(s), ou

c) on condense des dérivés d'acide α-hydrazinoacétique de formule générale

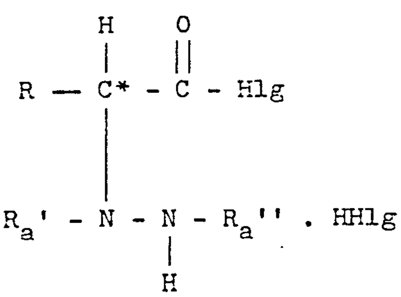

(V)

dans laquelle

R et l'astérisque (*) sont tels que définis plus haut,

$R_a'$ représente l'hydrogène, un groupe alkyle de 1 à 3 atomes de carbone ou un groupe protecteur usuel,

$R_a''$ représente l'hydrogène ou un groupe protecteur usuel, au moins un des symboles $R_a'$ et $R_a''$ ne représentant pas un groupe protecteur, et

Hlg représente le chlore ou le brome,

sous la forme des épimères séparés ou de mélanges de ceux-ci, avec l'acide 6-aminopénicillanique, tel quel ou sous forme de sel ou sous forme d'esters, le groupe ester n'ayant éventuellement qu'une fonction usuelle de groupe protecteur, et on formyle, en milieu anhydre, avec l'anhydride mixte de l'acide acétique et de l'acide formique, les hydrazinométhylpénicillines ainsi obtenues, de formule générale

(Voir formule, page 30)

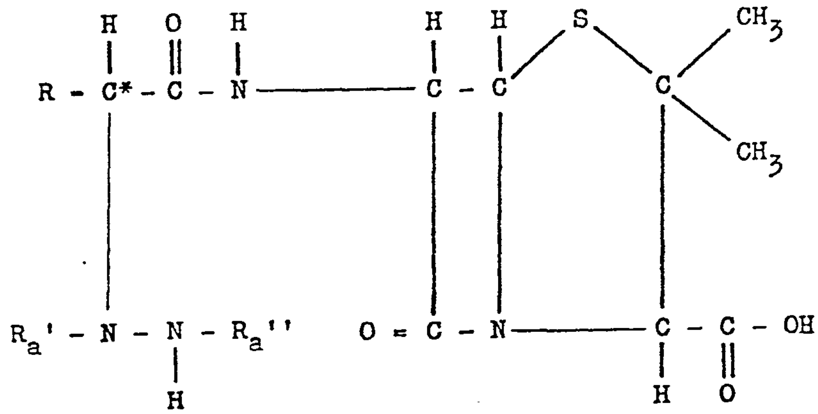

(II)

dans laquelle R, l'astérique (*), $R_a'$ et $R_a''$ sont tels que définis plus haut, et on élimine le ou les groupe(s) protecteur(s) éventuellement présent(s),
après quoi, de manière en elle-même connue, on convertit éventuellement les dérivés formylés d'hydrazinométhylpénicillines ainsi obtenus, de la formule générale I, en sels d'addition d'acide pharmaceutiquement utilisables, au moyen d'acides, ou on les estérifie, ou éventuellement, on convertit les sels d'addition d'acide des dérivés formylés d'hydrazinométhylpénicillines de la formule générale I, ainsi obtenus, en dérivés formylés libres d'hydrazinométhylpénicillines de la formule générale I ou en d'autres sels, ou éventuellement on convertit les esters de dérivés formylés d'hydrazinométhylpénicillines de la formule générale I, ainsi obtenus, en dérivés formylés libres d'hydrazinométhylpénicillines de la formule générale I et/ou éventuellement, on dédouble en épimères les mélanges d'épimères de dérivés formylés d'hydrazinométhylpénicillines de la formule générale I, ainsi obtenus, ou on racémise les épimères de dérivés formylés d'hydrazinométhylpénicillines de la formule générale I, ainsi obtenus.

2. Procédé selon la revendication 1, caractérisés par le fait qu'on emploie comme radical alkyle que peut représenter R' un tel contenant un ou deux atome(s) de carbone, en particulier un seul atome de carbone.

3. Procédé selon la revendication 1 ou 2, caractérisé par le fait qu'on prépare l'acide (R)-6β-[α-(2'-formyl-1'-méthylhydrazino)-phénylacétamido]-pénicillanique.

4. Procédé selon la revendication 1 ou 2, caractérisé par le fait qu'on prépare l'acide (R)-6β-[α-(1',2'-diformylhydrazino)-phénylacétamido]-pénicillanique.

5. Procédé selon la revendication 1 ou 2, caractérisé par le fait qu'on prépare l'acide (R)-6β-[α(2'-formylhydrazino)-phénylacétamido]-pénicillanique.

6. Procédé selon la revendication 1 ou 2, caractérisé par le fait qu'on prépare le (R)-6β-[α-(2'-formylhydrazino)-phénylacétamido]-pénicillanate de 1''-(éthoxycarbonyloxy)-éthyle.